(19) Europäisches Patentamt / European Patent Office / Office européen des brevets

(11) **EP 2 578 243 B1**

(12) **EUROPEAN PATENT SPECIFICATION**

(45) Date of publication and mention
of the grant of the patent:
**06.09.2017 Bulletin 2017/36**

(51) Int Cl.:
*A61L 9/22* *(2006.01)*    *B03C 3/02* *(2006.01)*
*B03C 3/155* *(2006.01)*    *B03C 3/41* *(2006.01)*
*B03C 3/68* *(2006.01)*

(21) Application number: **11789430.3**

(22) Date of filing: **30.05.2011**

(86) International application number:
**PCT/JP2011/002998**

(87) International publication number:
**WO 2011/152016 (08.12.2011 Gazette 2011/49)**

(54) **DEVICE FOR MICROBE AND VIRUS CAPTURE AND INACTIVATION**

VORRICHTUNG ZUR ERFASSUNG UND DEAKTIVIERUNG VON MIKROBEN UND VIREN

DISPOSITIF DE CAPTURE ET D'INACTIVATION DE MICROBES ET DE VIRUS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.12.2010  JP 2010284547
02.06.2010  JP 2010126727**

(43) Date of publication of application:
**10.04.2013  Bulletin 2013/15**

(73) Proprietor: **Mitsubishi Electric Corporation
Tokyo 100-8310 (JP)**

(72) Inventors:
• **TANIMURA, Yasuhiro
Tokyo 100-8310 (JP)**
• **OTA, Koji
Tokyo 100-8310 (JP)**

• **NOMURA, Akane
Tokyo 100-8310 (JP)**
• **SAIKI, Ayumi
Tokyo 100-8310 (JP)**

(74) Representative: **J A Kemp
14 South Square
Gray's Inn
London WC1R 5JJ (GB)**

(56) References cited:
EP-A1- 1 980 317        JP-A- 1 249 145
JP-A- 2 035 947         JP-A- H01 249 145
JP-A- 2002 095 996      JP-A- 2005 304 762
JP-A- 2005 304 762      JP-A- 2006 043 550
JP-A- 2006 043 550      JP-A- 2009 519 819
JP-A- 2010 022 998      JP-A- 2010 022 998
JP-A- 2010 029 865      JP-A- 2010 029 865
US-A- 5 403 383         US-A1- 2008 170 971

**Description**

Technical Field

The present invention relates to an apparatus and method for capture and inactivation of microbes and viruses, the apparatus and method being capable of capturing and inactivating a microbe and/or a virus suspended in a space.

Background Art

There have been airborne microbe/virus removal apparatuses for removing microbes and viruses suspended in a space. Such an airborne microbe/virus removal apparatus is disclosed which includes a corona charging unit, a high-voltage electrode, a filter, and an electrode in contact with the filter arranged in that order from a windward side to cancel out the effect of charge accumulation during operation so that high removal performance can be provided throughout a long life (refer to Patent Literature 1, for example).

Another airborne microbe/virus removal apparatus is disclosed which includes a pre-filter, a charging unit, a photocatalytic filter, an ultraviolet lamp, a virus capture filter, and an electrostatic filter arranged in that order from a windward side to enable functions of capturing and inactivating pathogenic viruses, such as an influenza virus, to be maintained for a long time (refer to Patent Literature 2, for example).

Citation List

Patent Literature

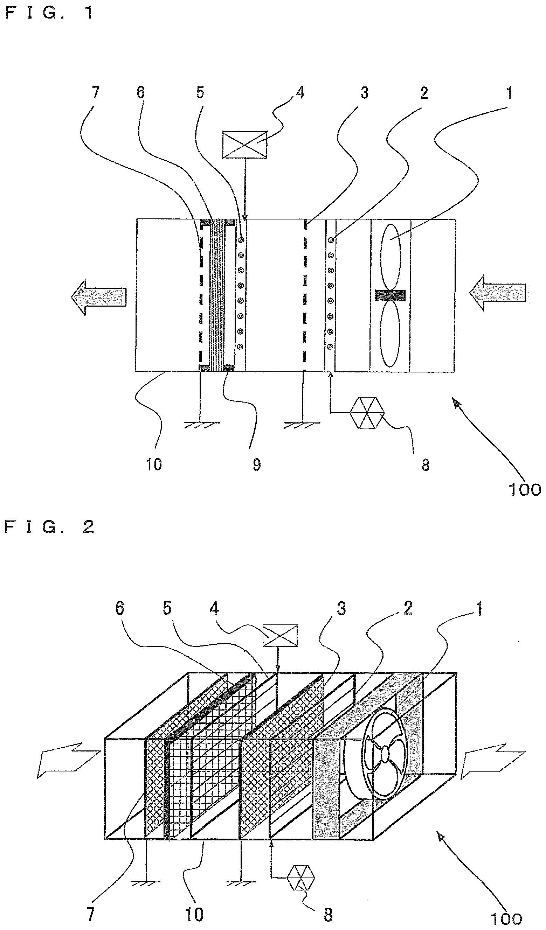

Patent Literature 1: Japanese Unexamined Patent Application Publication (Translation of PCT Application) No. 2007-512131 (p. 7, l. 17 to p. 10, l. 30, Fig. 1, for example)
Patent Literature 2: Japanese Unexamined Patent Application Publication No. 11-188214 (p.7, l. 41 to p. 8, l. 51, Fig. 1, for example)

US 2008/170971 A1 discloses the preamble to claim 1. JP 1249145 A, JP 2010 022998 A1, JP 2010029865, JP 2006 043550 A, EP 1980317 A1, and JP 2005 304762 A are relevant to the background art.

Summary of Invention

Technical Problem

In the airborne microbe/virus removal apparatus disclosed in Patent Literature 1, microbes and viruses deposited on the filter are again scattered by application of an electric field. Disadvantageously, this lowers the advantage of capturing airborne microbes and airborne viruses in the airborne microbe/virus removal apparatus disclosed in Patent Literature 1. Furthermore, the airborne microbe/virus removal apparatus disclosed in Patent Literature 1 requires maintenance, such as filter cleaning, in order to prevent microbes and viruses captured by the filter from growing.

The airborne microbe/virus removal apparatus disclosed in Patent Literature 2 includes three filters, namely, the photocatalytic filter, the water drop type filter, and the electrostatic filter. Accordingly, this arrangement leads to an increase in pressure loss in the airborne microbe/virus removal apparatus disclosed in Patent Literature 2. Disadvantageously, for example, energy loss or noise may be caused.

The present invention has been made to overcome the above-described disadvantages and provides an apparatus and method for capture and inactivation of microbes and viruses, the apparatus and method being capable of stably removing a microbe and/or a virus and achieving a reduction in pressure loss. Solution to Problem

The present invention is defined by the appended claims.

Advantageous Effects of Invention

The apparatus and method for capture and inactivation of microbes and viruses according to the present invention enable capture of microbes and/or viruses suspended in air with low pressure loss such that the microbes and/or viruses suspended in the air are charged and then captured, and enable inactivation of the captured viruses by discharge. Advantageously, a portion where the microbes and/or viruses are captured can be kept in a clean state at all times.

Brief Description of Drawings

[Fig. 1] Fig. 1 is a sectional view illustrating a longitudinal section of a schematic configuration of an apparatus for capture and inactivation of microbes and viruses according to Example1.

[Fig. 2] Fig. 2 is a perspective view of the schematic configuration of the apparatus for capture and inactivation of microbes and viruses according to Example 1.

[Fig. 3] Fig. 3 is a flowchart illustrating the flow of a method for capture and inactivation of microbes and viruses, the method being executed by the apparatus for capture and inactivation of microbes and viruses according to Example 1.

[Fig. 4] Fig. 4 is a graph of the relationship between an electric field strength (kV/cm) and a transient virus capture rate (%), the relationship being examined.

[Fig. 5] Fig. 5 is a graph of the effect of the polarity of a voltage applied to a charging-unit high-voltage electrode on the transient virus capture rate (%) and the concentration (ppm) of ozone generated, the effect being examined.

[Fig. 6] Fig. 6 is a graph of the effect of the polarities of voltages applied to the charging-unit high-voltage electrode and a capturing/inactivating-unit high-voltage electrode on the transient virus capture rate (%), the effect being examined.

[Fig. 7] Fig. 7 is a graph of the effects of the charging-unit high-voltage electrode and air velocity on the transient virus capture rate, the effects being examined.

[Fig. 8] Fig. 8 is a graph of the comparison in the survival rate of captured viruses between ozone processing and plasma processing.

[Fig. 9] Fig. 9 is a sectional view illustrating a longitudinal section of a schematic configuration of an apparatus for capture and inactivation of microbes and viruses according to Example 2.

[Fig. 10] Fig. 10 is a sectional view illustrating a longitudinal section of a schematic configuration of an apparatus for capture and inactivation of microbes and viruses according to Example 3.

[Fig. 11] Fig. 11 is a sectional view illustrating a longitudinal section of a schematic configuration of an apparatus for capture and inactivation of microbes and viruses according to Example 4.

[Fig. 12] Fig. 12 is a sectional view illustrating a longitudinal section of a schematic configuration of an apparatus for capture and inactivation of microbes and viruses according to Example 5.

[Fig. 13] Fig. 13 is a sectional view illustrating a longitudinal section of a schematic configuration of an apparatus for capture and inactivation of microbes and viruses according to Example 6.

[Fig. 14] Fig. 14 is a sectional view illustrating a longitudinal section of a schematic configuration of an apparatus for capture and inactivation of microbes and viruses according to Example 7.

[Fig. 15] Fig. 15 is a sectional view illustrating a longitudinal section of a schematic configuration of an apparatus for capture and inactivation of microbes and viruses according to a modification of Example 7.

[Fig. 16] Fig. 16 is a sectional view illustrating a longitudinal section of a schematic configuration of an apparatus for capture and inactivation of microbes and viruses according to Embodiment 1 of the present invention.

[Fig. 17] Fig. 17 is a sectional view illustrating a longitudinal section of a schematic configuration of an apparatus for capture and inactivation of microbes and viruses according to Example 8.

[Fig. 18] Fig. 18 is a sectional view illustrating a longitudinal section of a schematic configuration of an apparatus for capture and inactivation of microbes and viruses according to Example 9.

[Fig. 19] Fig. 19 is a sectional view illustrating a longitudinal section of a schematic configuration of an apparatus for capture and inactivation of microbes and viruses according to Example 10.

[Fig. 20] Fig. 20 includes schematic views illustrating a structure of a hydrophilic filter in the apparatus for capture and inactivation of microbes and viruses according to Example 1.

[Fig. 21] Fig. 21 is a sectional view illustrating a longitudinal section of a schematic configuration of an apparatus for capture and inactivation of microbes and viruses according to a modification of Example 5.

[Fig. 22] Fig. 22 is a sectional view illustrating a longitudinal section of a schematic configuration of an apparatus for capture and inactivation of microbes and viruses according to Example 11.

[Fig. 23] Fig. 23 is a sectional view illustrating a longitudinal section of a schematic configuration of an apparatus for capture and inactivation of microbes and viruses according to a modification of Example 11.

[Fig. 24] Fig. 24 is a sectional view illustrating a longitudinal section of a schematic configuration of an apparatus for capture and inactivation of microbes and viruses according to Example 12.

[Fig. 25] Fig. 25 is a diagram illustrating the waveform of a voltage applied to the charging-unit high-voltage electrode.

[Fig. 26] Fig. 26 is a sectional view illustrating a longitudinal section of a schematic configuration of an apparatus for capture and inactivation of microbes and viruses according to Example 13.

[Fig. 27] Fig. 27 is a graph illustrating a change in influenza virus survival rate with varying temperature and humidity.

[Fig. 28] Fig. 28 is a graph illustrating features of the rate of particle capture depending on particle size.

[Fig. 29] Fig. 29 is a sectional view illustrating a longitudinal section of a schematic configuration of an apparatus for capture and inactivation of microbes and viruses according to a modification of Example 13, the apparatus further including sensors and a controller.

[Fig. 30] Fig. 30 is a graph of the effect of the distance between the capturing/inactivating-unit high-voltage electrode and the hydrophilic filter on the concentration of ozone gas generated upon processing with plasma and processing time required to achieve a virus inactivation rate of 99%.

[Fig. 31] Fig. 31 is a sectional view illustrating a longitudinal section of a schematic configuration of an apparatus for capture and inactivation of microbes and viruses according to Example 6A.

[Fig. 32] Fig. 32 is a sectional view illustrating a longitudinal section of a schematic configuration of an apparatus for capture and inactivation of microbes and viruses according to Example 15.

[Fig. 33] Fig. 33 is a flowchart illustrating the flow of a method for capture and inactivation of microbes and viruses, the method being executed by the apparatus for capture and inactivation of microbes and viruses according to Example 15.

[Fig. 34] Fig. 34 is a sectional view illustrating a longitudinal section of a schematic configuration of an apparatus for capture and inactivation of microbes and viruses according to Example 16.

[Fig. 35] Fig. 35 is a graph of the effect of moving air on virus inactivation with plasma.

[Fig. 36] Fig. 36 is a sectional view illustrating a longitudinal section of a schematic configuration of an apparatus for capture and inactivation of microbes and viruses according to Example 17.

[Fig. 37] Fig. 37 is a block diagram illustrating the configuration of a charging/inactivating high voltage power supply.

Description of Embodiments

Examples outside the scope of the invention and Embodiments of the present invention will be described below with reference to the drawings.

Example 1

Fig. 1 is a sectional view illustrating a longitudinal section of a schematic configuration of an apparatus (hereinafter, referred to as the "apparatus 100") for capture and inactivation of microbes and viruses according to Example 1. Fig. 2 is a perspective view illustrating of the schematic configuration of the apparatus 100. The configuration and operation of the apparatus 100 will be described with reference to Figs. 1 and 2. Note that the dimensional relationship among components in Fig. 1 and the subsequent figures may be different from the actual one. Furthermore, the flow of air is indicated by arrows in Figs. 1 and 2.

The apparatus 100 is configured to capture microbes and viruses (hereinafter, also referred to as "airborne microorganisms) suspended in a space and inactivate the captured airborne microorganisms. The apparatus 100 includes an air path housing 10, an air-sending device 1, a charging-unit high-voltage electrode (first high-voltage application electrode) 2, a charging-unit ground electrode (first counter electrode) 3, a capturing/inactivating-unit high-voltage electrode (second high-voltage application electrode) 5, a hydrophilic filter 6, and a capturing/inactivating-unit ground electrode (second counter electrode) 7 such that the components are arranged in the air path housing 10 in that order from a windward (upstream) side.

The air-sending device 1 is configured to introduce air into the air path housing 10. The charging-unit high-voltage electrode 2 is an electrode including many stretched wires having a diameter in the range of, for example, approximately 0.1 mm to approximately 0.3 mm and is configured to be applied with a high voltage from a high voltage power supply 8 connected to the electrode. The charging-unit ground electrode 3 is an electrode made of, for example, metal mesh and is connected to ground. The charging-unit high-voltage electrode 2 and the charging-unit ground electrode 3 constitute a charging unit. While Example 1 has been described on the assumption that the charging-unit ground electrode 3 functions as a first counter electrode, it is only required that a voltage is applied between the charging-unit high-voltage electrode 2 and the charging-unit ground electrode 3. The charging-unit ground electrode 3 does not necessarily have to be grounded. Furthermore, if the charging-unit high-voltage electrode 2 is made of ribbon which has a rectangular or similar shaped cross-section having a sectional area of 0.1 mm x 0.5 mm (and which has a thickness in the range of 0.1 mm to 0.3 mm), the same advantages will be offered. In this case, more efficient charging is achieved in such an arrangement that a surface defined by short sides (0.1 mm) of the sectional area faces the charging-unit ground electrode 3. Furthermore, advantageously, the influence of disconnection due to electrode wear-out caused by sputtering upon discharge can be reduced.

The capturing/inactivating-unit high-voltage electrode 5 is an electrode including many stretched wires having a diameter in the range of, for example, approximately 0.1 mm to 0.3 mm and is configured to be applied with a high voltage from a variable high voltage power supply 4 connected to the electrode. The capturing/inactivating-unit ground electrode 7 is an electrode made of, for example, metal mesh and is connected to the ground. While Embodiment 1 has been described on the assumption that the capturing/inactivating-unit ground electrode 7 functions as a second counter electrode, it is only required that a voltage is applied between the capturing/inactivating-unit high-voltage electrode 5 and the capturing/inactivating-unit ground electrode 7. The capturing/inactivating-unit ground electrode 7 does not necessarily have to be grounded. Furthermore, if the capturing/inactivating-unit high-voltage electrode 5 is made of ribbon which has a rectangular or similar shaped cross-section having a sectional area of 0.1 mm x 0.5 mm (and which has a thickness of 0.1 mm), the same advantages will be offered. In this case, a surface defined by short sides (0.1 mm) of the sectional area may be allowed to face the charging-unit ground electrode 3.

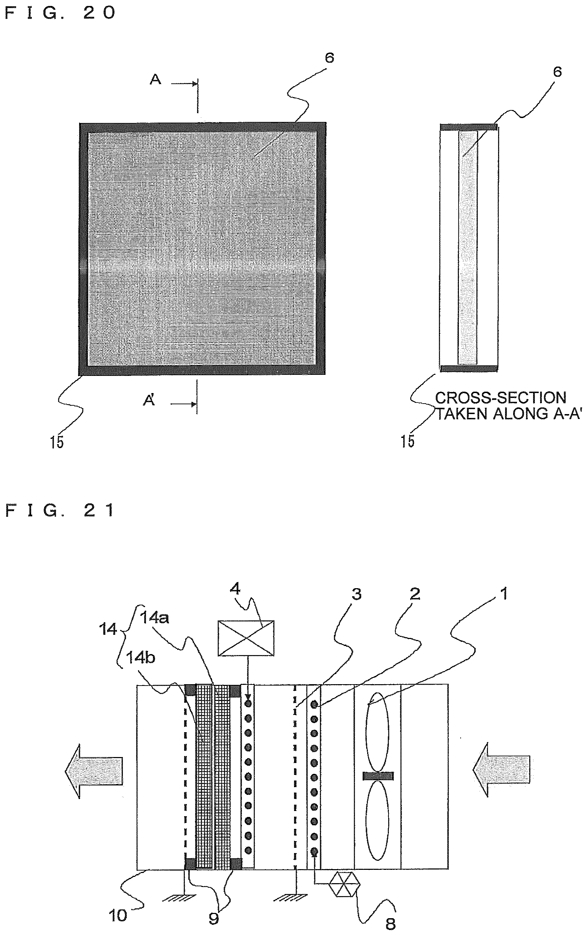

The hydrophilic filter 6 is insulated by bushings 9 such that the filter is sandwiched by the capturing/inactivating-unit high-voltage electrode 5 and the capturing/inactivating-unit ground electrode 7 paired with each other. The capturing/inactivating-unit high-voltage electrode 5, the hydrophilic filter 6, and the capturing/inactivating-unit ground electrode 7 constitute a capturing/inactivating unit. The variable high voltage power supply 4 is capable of applying a voltage at one of at least two levels to the capturing/inactivating-unit high-voltage electrode 5. Note that the hydrophilic filter 6 may be housed in an insulating frame (frame 15) as illustrated in Fig. 20, instead of being insulated by the bushings 9.

The above configuration enables the hydrophilic filter 6, which is sandwiched by the capturing/inactivating-unit high-voltage electrode 5 and the capturing/inactivating-unit ground electrode 7 and is isolated and grounded, to exhibit dielectric behavior, or polarization. An electrostatic field is produced on one surface of the hydrophilic filter 6. Accordingly, airborne microorganisms charged, or with charges applied by the charging unit composed of the charging-unit high-voltage electrode 2 and the charging-unit ground electrode 3 are attracted to the electric field produced on the surface of the hydrophilic filter 6, so that the airborne microorganisms come into collision with the hydrophilic filter 6. Furthermore, water suspended with the airborne microorganisms collides with the hydrophilic filter 6, so that the water adheres to the hydrophilic filter 6. Consequently, microbes and viruses are prevented from being scattered again. The microbes and viruses captured by the hydrophilic filter 6 are inactivated by discharge products produced by discharge through the capturing/inactivating-unit high-voltage electrode 5.

As described above, the hydrophilic filter 6 constitutes the capturing unit in the apparatus 100. Subjecting the hydrophilic filter 6 to electrostatic induction efficiently induces charged airborne microorganisms, so that the microorganisms collide with the surface of the hydrophilic filter 6 and the airborne microorganisms subjected to collision can be held with water. Advantageously, the apparatus 100 can capture airborne microorganisms with low pressure loss and can also prevent captured microbes and viruses from being scattered again.

Note that the hydrophilic filter 6 may be of any kind capable of absorbing water (atomized water) subjected to collision. If the hydrophilic filter 6 is of a type that prevents formation of water droplets on the surface of the filter upon collision with water, the water held on the surface can be prevented from being scattered again. Thus, high capturing performance can be maintained.

An operation of the apparatus 100 will now be described.

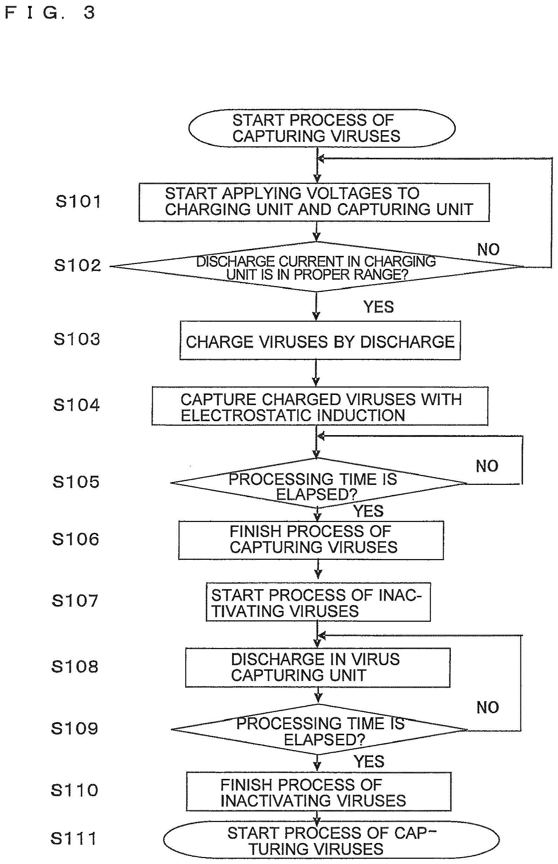

Fig. 3 is a flowchart illustrating the flow of a method for capture and inactivation of microbes and viruses, the method being executed by the apparatus 100. The apparatus 100 has a feature in that part for capturing airborne microorganisms and part for inactivating the captured airborne microorganisms are standardized. Specifically, since the apparatus 100 is capable of executing a process of capturing microbes and viruses and a process of inactivating the captured microbes and viruses in a sequential order, the microbes and viruses can be removed with high efficiency.

When the apparatus 100 starts operation, the air-sending device 1 is activated. The high voltage power supply 8 applies a high voltage to the charging-unit high-voltage electrode 2 and the variable high voltage power supply 4 applies a high voltage to the capturing/inactivating-unit high-voltage electrode 5 (step S101). Thus, discharge occurs between the charging-unit high-voltage electrode 2 and the charging-unit ground electrode 3, so that discharge current flows into the charging-unit ground electrode 3. A current flowing into the charging-unit ground electrode 3 is measured by a current determining unit provided for, for example, a control board (not illustrated). The measured current is compared with a reference current previously set by the current determining unit (step S102). If there is no problem, the process proceeds to the next step (YES in step S102).

If the measured current is lower than the reference current, the voltage to be applied to the charging-unit high-voltage electrode 2 is raised. If the measured current is higher than the reference current, the voltage to be applied to the charging-unit high-voltage electrode 2 is lowered (step S103). Whether airborne microbes and viruses are efficiently charged in this manner at all times is determined (step S104). Upon start of the step (step S103) of charging microbes and viruses by discharge and the step (step S104) of capturing the charged microbes and viruses by electrostatic induction, a timer is activated to count processing time of these steps (step S105).

When the processing time of these steps reaches reference time (YES in step S105), the application of the high voltage to the charging-unit high-voltage electrode 2 is stopped and the application of the high voltage to the capturing/inactivating-unit high-voltage electrode 5 is also stopped. The air-sending device 1 is then stopped. The series of steps (i.e., the process of capturing microbes and viruses) is finished (step S106).

The process of inactivating microbes and viruses is then started. The variable high voltage power supply 4 applies a high voltage to the capturing/inactivating-unit high-voltage electrode 5. Thus, discharge occurs between the capturing/inactivating-unit high-voltage electrode 5 and the capturing/inactivating-unit ground electrode 7, so that discharge current flows into the capturing/inactivating-unit ground electrode 7. At this time, a current flowing into the capturing/inactivating-unit ground electrode 7 is measured by the current measuring unit. The measured current is compared with a reference current previously set by the current determining unit. If there is no problem, the inactivating process is started (step S107).

If the measured current is lower than the reference current, the voltage to be applied to the capturing/inactivating-unit high-voltage electrode 5 is raised. If the measured current is higher than the reference current, the voltage to be applied to the capturing/inactivating-unit high-voltage electrode 5 is lowered (step S108). Whether airborne microbes and viruses are efficiently inactivated in this manner at all times is determined. Upon start of the process of inactivating microbes and viruses by discharge (steps S107 and S108), the timer is activated to count processing time of these steps (step S109).

When the processing time of these steps reaches reference time (YES in step S109), the application of the high voltage to the capturing/inactivating-unit high-voltage electrode 5 is stopped. The inactivating process is finished (step S110). After that, the process of charging and capturing microbes and viruses is again started (step S111). The above-described operation is repeated.

As described above, the apparatus 100 executes the step of charging airborne microorganisms (the step of allowing airborne microorganisms to be charged), the step of capturing the charged airborne microorganisms with the hydrophilic filter 6 subjected to electrostatic induction, and the step of inactivating the airborne microorganisms captured by the hydrophilic filter 6 with plasma. Advantageously, the portion (hydrophilic filter 6) capturing the airborne microorganisms can be kept in a clean state at all times. Accordingly, the air in the space (such as a living space) where the apparatus 100 is installed can also be kept in a clean state at all times.

Low pressure loss and highly efficient capture due to charging by corona discharge and electrostatic induction in the hydrophilic filter 6, as features of the apparatus 100, will now be described. Table 1 illustrates the comparison in pressure loss (Pa) and transient virus capture rate (%) among the system of the apparatus 100 and related-art filtering systems.

[Table 1]

|  | System of this application | HEPA filter | Normal filter |
| --- | --- | --- | --- |
| Pressure loss [Pa] (at 1 m/s) | 10 | 150 | 10 |
| Transient virus capture rate [%] | 95 | 99.9 | 5 or less |

Table 1 demonstrates that the system of the apparatus 100, namely, the electrostatic induction system using the hydrophilic filter 6 had a pressure loss of approximately 10 Pa, which is equal to that in a normal filter, in the flow of moving air at a linear velocity of 1 m/s. The transient virus capture rate at that time was approximately 95%, which is markedly higher than a transient virus capture rate of 5% in the normal filter. This may be attributed to that static electricity enables viruses to collide with the filter with efficiency and the absorbability of water prevents the viruses subjected to collision from being scattered again. Furthermore, it was found that the transient virus capture rate in the HEPAfilter (high efficiency particulate air filter) is higher than that in the system of the apparatus 100 but pressure loss in the filter is significantly higher than that in the system.

The above facts indicate that the use of the system of the apparatus 100 enables charging by corona charge and electrostatic induction in the hydrophilic filter 6 to achieve the same level of transient virus capture rate as that in the HEPA filter while keeping the same level of pressure loss as that in the normal filter.

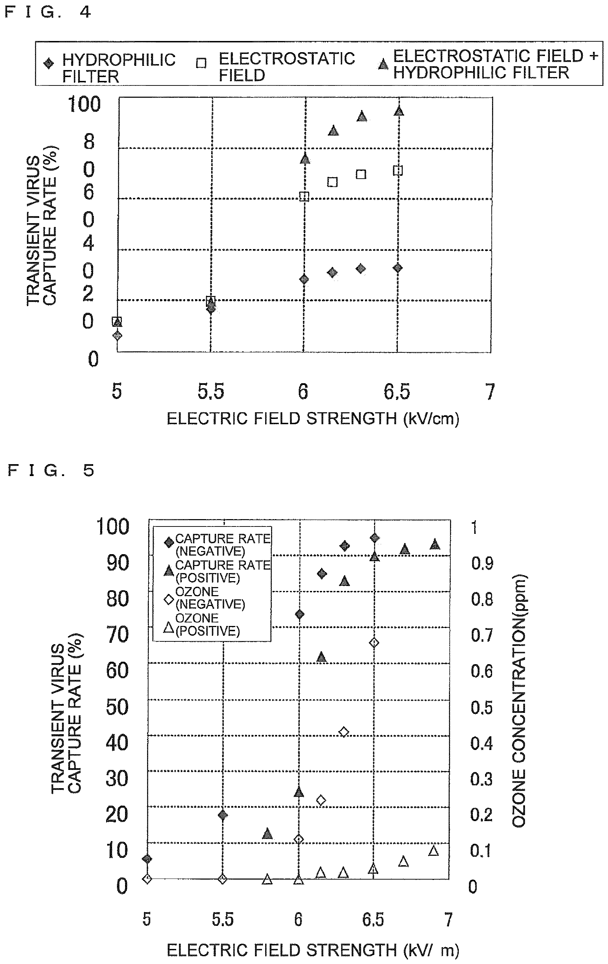

The effect of electrostatic induction in the hydrophilic filter 6, as the feature of the apparatus 100, on highly efficient virus capture will now be described. Fig. 4 is a graph of the relationship between the strength (kV/cm) of the electric field between the capturing/inactivating-unit high-voltage electrode 5 and the hydrophilic filter 6 and the transient virus capture rate (%), the relationship being examined. In Fig. 4, the axis of abscissas indicates the electric field strength and the axis of ordinates indicates the transient virus capture rate.

Referring to Fig. 4, in the case where the hydrophilic filter 6 was not subjected to electrostatic induction, the transient virus capture rate was approximately 30% (indicated by solid black rectangles in Fig. 4) though viruses were charged by corona discharge. In the case where a filter was subjected to electrostatic induction, the transient virus capture rate was increased to 70% (indicated by open rectangles in Fig. 4). Furthermore, in the case where the hydrophilic filter 6 was subjected to electrostatic induction, the transient virus capture rate was increased to 95% (indicated by solid black triangles in Fig. 4).

The above facts indicate that it is very important to subject the hydrophilic filter 6 to electrostatic induction. Thus, it is apparent that the hydrophilic filter 6 has to be subjected to electrostatic induction in order to achieve capture at or above 90%, at which it is generally determined that the advantage of removing microbes and viruses is offered.

Fig. 5 is a graph of the effect of the polarity of a voltage applied to the charging-unit high-voltage electrode 2 on the transient virus capture rate (%) and the concentration (ppm) of ozone generated, the effect being examined. In Fig. 5, the axis of abscissas indicates the strength (kV/cm) of the electric field between the charging-unit high-voltage electrode 2 and the charging-unit ground electrode 3, the left side of the axis of ordinates indicates the transient virus capture rate, and the right side thereof indicates the concentration of ozone generated.

Referring to Fig. 5, when a negative voltage was applied to the charging-unit high-voltage electrode 2, a transient virus capture rate of 95% was achieved with a lower applied voltage (indicated by a solid black rectangle in Fig. 5). Furthermore, it was found that a positive voltage was preferably applied in order to achieve the concentration of ozone at or below 0.1 ppm at a transient virus capture rate of 95% (indicated by an open triangle in Fig. 5).

The above facts indicate that, in the case where the apparatus 100 is adapted for use in air-conditioning equipment, it is preferable to charge viruses with positive voltage application to the electrode which enables maintaining a high transient virus capture rate while keeping an amount of ozone generated lower.

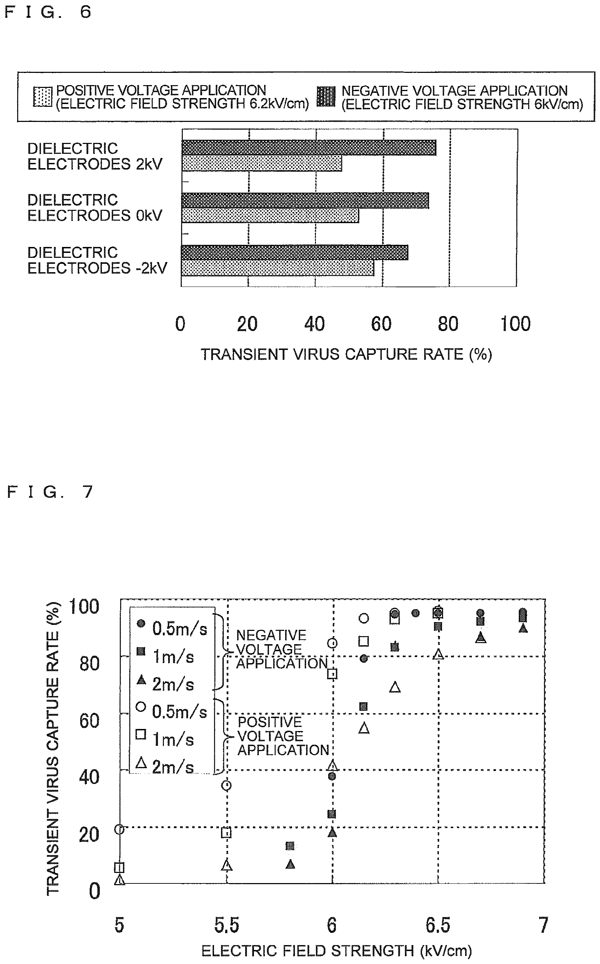

Fig. 6 is a graph of the effect of the polarities of voltages applied to the charging-unit high-voltage electrode 2 and the capturing/inactivating-unit high-voltage electrode 5 on the transient virus capture rate (%), the effect being examined. Referring to Fig. 6, in the case where a positive voltage was applied to the charging-unit high-voltage electrode 2, the transient virus capture rate was increased when the voltage applied to the capturing/inactivating-unit high-voltage electrode 5 was negative. In the case where a negative voltage was applied to the charging-unit high-voltage electrode 2, the transient virus capture rate was increased when the voltage applied to the capturing/inactivating-unit high-voltage electrode 5 was positive.

The above facts indicate that allowing the voltages applied to the charging-unit high-voltage electrode 2 and the capturing/inactivating-unit high-voltage electrode 5 to have opposite polarities increases the transient virus capture rate.

Fig. 7 is a graph of the effect of the polarity of a voltage applied to the charging-unit high-voltage electrode 2 and air velocity on the transient virus capture rate, the effect being examined. In Fig. 7, the axis of abscissas indicates the strength (kV/cm) of the electric field between the charging-unit high-voltage electrode 2 and the charging-unit ground electrode 3 and the axis of ordinates indicates the transient virus capture rate (%). Solid black marks indicate values obtained upon application of a negative voltage to the charging-unit high-voltage electrode 2 and open marks indicate values obtained upon application of a positive voltage to the charging-unit high-voltage electrode 2. Fig. 7 demonstrates that, in the case where the absolute value of an applied voltage was 6 kV or 6.3 kV, the degree of variation in transient virus capture rate upon change in air velocity was large when the negative voltage was applied to the charging-unit high-voltage electrode 2.

The above facts indicate that stable virus removal is achieved when a positive voltage is applied to the charging-unit high-voltage electrode 2 in a system in which the air velocity changes.

Inactivation of viruses captured with the hydrophilic filter 6 by discharge will now be described, the inactivation being the second feature of the apparatus 100. Typically, viruses are not inactivated by merely applying voltages to electrodes such that the electrodes are polarized. The apparatus 100 is therefore designed to inactivate viruses using discharge products derived from discharge caused by voltage application.

In investigating the electric field strengths and the polarities of applied voltages affected on the concentration (ppm) of ozone gas generated as one of discharge products, it can be seen from Fig. 5 that the concentration of ozone gas upon negative voltage application was higher than that upon positive voltage application at the same electric field strength. This fact indicates that negative voltage application is preferable in order to increase the efficiency of virus inactivation.

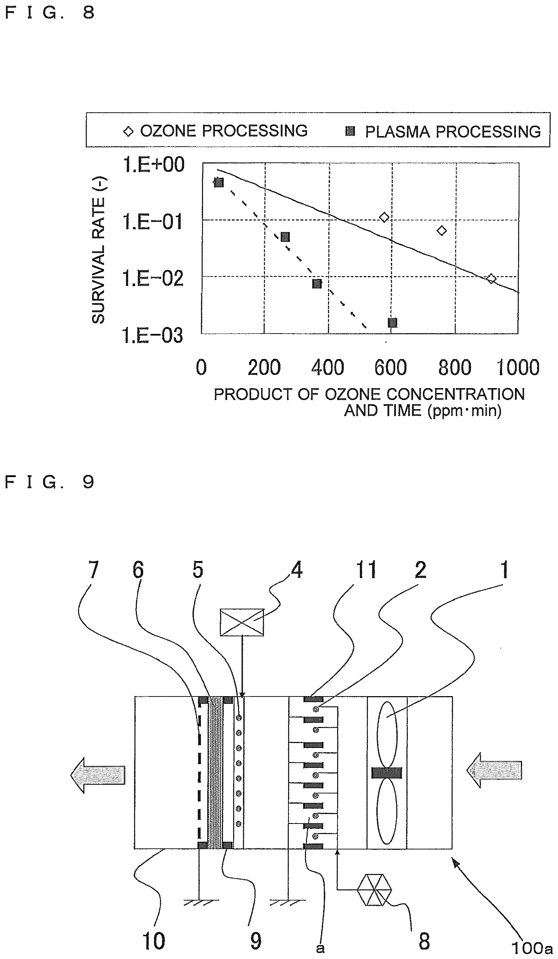

Fig. 8 is a graph of the comparison in virus survival rate between processing captured viruses with only the ozone gas and processing (plasma processing) captured viruses with other discharge products in addition to the ozone gas. In Fig. 8, the axis of abscissas indicates the product (ppm·min) of the concentration of ozone and time and the axis of ordinates indicates the survival rate (-). As illustrated in Fig. 8, even when ozone processing and plasma processing were performed at the same concentration of ozone, processing viruses in a plasma field achieved inactivation in a shorter time, probably because the viruses were inactivated by, for example, electrons, radicals, and ions in plasma, since captured viruses were exposed to the plasma field.

Accordingly, if the apparatus is designed such that the hydrophilic filter 6 for capturing viruses is placed in the plasma field, viruses can be inactivated in a short time. Advantageously, since the time required for inactivation can be reduced and the time required to capture airborne microorganisms can be extended, the apparatus 100 can remove the airborne microorganisms with higher efficiency.

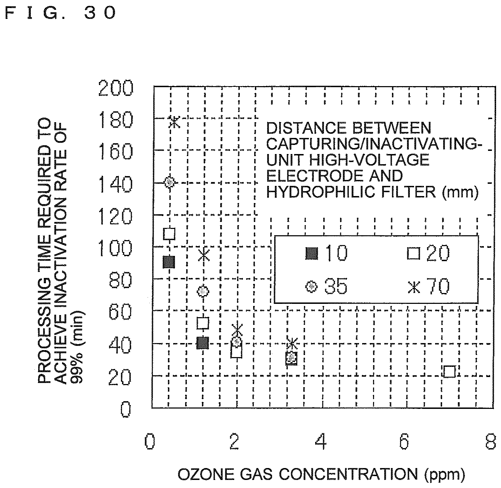

Fig. 30 is a graph of the effect of the distance between the capturing/inactivating-unit high-voltage electrode 5 and the hydrophilic filter 6 on the concentration of ozone gas generated upon plasma processing and the processing time required to achieve a virus inactivation rate of 99%. The axis of abscissas indicates the concentration of ozone gas (plasma processing) and the axis of ordinates indicates the processing time required to achieve a virus inactivation rate of 99%.

Fig. 30 demonstrates that as the distance between the capturing/inactivating-unit high-voltage electrode 5 and the hydrophilic filter 6 was shorter, particularly, 20 mm or less, the processing time was dramatically shorter, probably because the shorter distance between the capturing/inactivating-unit high-voltage electrode 5 and the hydrophilic filter 6 increases the efficiency of virus inactivation by discharge products, such as radicals, excluding the ozone gas, having short lifetime produced by the capturing/inactivating-unit high-voltage electrode 5.

As described above, setting the distance between the hydrophilic filter 6 capturing viruses and the capturing/inactivating-unit high-voltage electrode 5 generating the plasma field at 20 mm or less can reduce the time required for virus inactivation. Consequently, the apparatus 100 can remove airborne microorganisms with higher efficiency.

Although a filter for removing dust in the air prior to charging airborne microorganisms is not described in Example 1, it is needless to say that placing the filter for removing dust prior to the entrance of air into the charging unit for charging airborne microorganisms results in more efficient virus capture. Furthermore, while Example 1 has been described with respect to the case where the air-sending device 1 is disposed on the windward side such that the air is forced to enter the virus capturing unit, it is needless to say that the same bactericidal effect can be obtained in an arrangement in which the air-sending device 1 is disposed on a leeward side so as to suck the air from the virus capturing unit.

Example 2

Fig. 9 is a sectional view illustrating a longitudinal section of a schematic configuration of an apparatus (hereinafter, referred to as the "apparatus 100a") for capture and inactivation of microbes and viruses according to Example 2. The configuration and operation of the apparatus 100a will be described with reference to Fig. 9. The difference between Example 2 and Example 1 will be mainly described. The same components as those in Example 1 are designated by the same reference numerals. The flow of air is indicated by arrows in Fig. 9.

The apparatus 100a according to Example 2 includes a charging unit positioned downwind of the air-sending device 1, the charging unit including the charging-unit high-voltage electrode 2 and a charging-unit ground electrode 11. Specifically, the apparatus 100a differs from the apparatus 100 according to Example 1 in the structure of the charging unit. The charging-unit high-voltage electrode 2 is the electrode including many stretched wires having a diameter in the range of approximately 0.1 mm to approximately 0.3 mm and is applied with a high voltage from the high voltage power supply 8 connected to the electrode. The charging-unit ground electrode 11 is an electrode made of, for example, a metal plate and is grounded.

This configuration offers the advantages described in Embodiment 1 and further enables a discharge space (space a in Fig. 9) defined by the charging-unit high-voltage electrode 2 and the charging-unit ground electrode 11 to be supplied with the whole amount of air introduced, thus efficiently charging airborne microorganisms. Accordingly, the apparatus 100a can maximize the microbe/virus capture rate of the capturing unit including the capturing/inactivating-unit high-voltage electrode 5, the hydrophilic filter 6, and the capturing/inactivating-unit ground electrode 7. Furthermore, if the charging-unit high-voltage electrode 2 is made of ribbon which has a rectangular or similar shaped cross-section having a sectional area of 0.1 mm x 0.5 mm (and which has a thickness in the range of 0.1 mm to 0.3 mm), the same advantages will be offered. In this case, more efficient charging is achieved in an arrangement in which a surface defined by short sides (0.1 mm) of the sectional area faces the charging-unit ground electrode 11. Furthermore, advantageously, the influence of disconnection due to electrode wear-out caused by sputtering upon discharge can be reduced.

Example 3

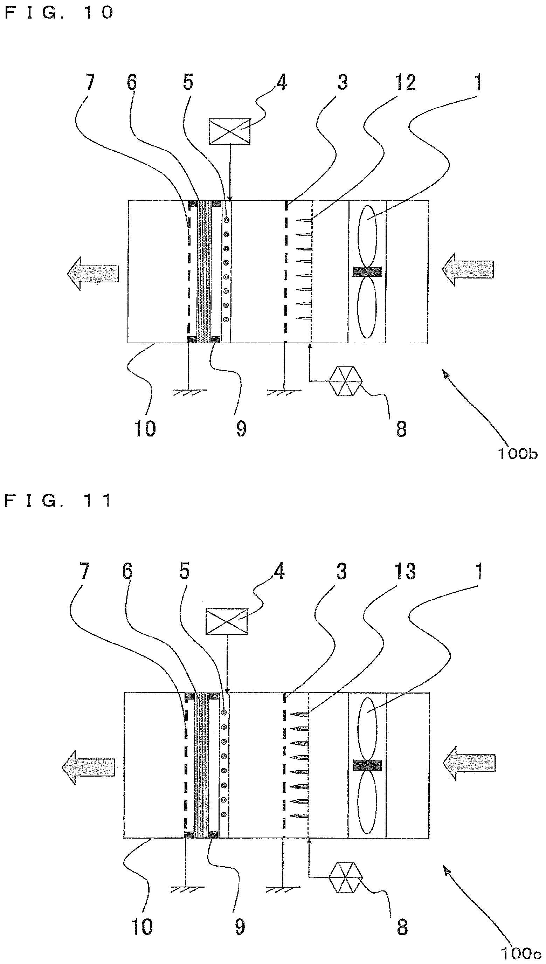

Fig. 10 is a sectional view illustrating a longitudinal section of a schematic configuration of an apparatus (hereinafter, referred to as the "apparatus 100b") for capture and inactivation of microbes and viruses according to Example 3. The configuration and operation of the apparatus 100b will be described with reference to Fig. 10. The difference between Example 3 and Examples 1 and 2 will be mainly described. The same components as those in Examples 1 and 2 are designated by the same reference numerals. The flow of air is indicated by arrows in Fig. 10.

While Example 1 relates to the configuration in which the charging-unit high-voltage electrode 2 including the wires is positioned on the windward side and the charging-unit ground electrode 3 including the metal mesh is positioned on the leeward side to charge airborne microorganisms, Example 3 relates to the configuration in which a charging-unit high-voltage electrode 12 is an electrode including a plurality of projections as illustrated in Fig. 10. For example, the projections may be attached to metal mesh or a plate, which permits the air to pass therethrough without pressure loss, by welding or the like to constitute the air the charging-unit high-voltage electrode 12 as illustrated in Fig. 10. According to a modification, a metal plate may be cut with a wire cutter or the like to form projections, thus constituting the charging-unit high-voltage electrode 12.

This configuration offers the advantages described in Example 1 and further prevents the charging-unit high-voltage electrode 12 from being damaged due to abnormal discharge caused by, for example, dust entering from the outside, so that stable discharge can be easily maintained.

Embodiment 4

Fig. 11 is a sectional view illustrating a longitudinal section of a schematic configuration of an apparatus (hereinafter, referred to as the "apparatus 100c") for capture and inactivation of microbes and viruses according to Example 4. The configuration and operation of the apparatus 100c will be described with reference to Fig. 11. The difference between Embodiment 4 and Examples 1 to 3 will be mainly described. The same components as those in Examples 1 to 3 are designated by the same reference numerals. The flow of air is indicated by arrows in Fig. 11.

While Example 3 relates to the configuration in which the charging-unit high-voltage electrode 12 is the electrode including the projections, Example 4 relates to the configuration in which a charging-unit high-voltage electrode 13 is an electrode including a plurality of projections and the surface of such a discharge electrode including the projections is coated with a catalyst. It is assumed that the surface of the discharge electrode is coated with a metal catalyst, such as silver (Ag), aluminum (Al), copper (Cu), or nickel (Ni).

This configuration offers the advantages described in Example 3 and further enables reduction of the amount of ozone generated while maintaining the efficiency of charging airborne microorganisms without lowering voltages to be applied. While Example 4 has been described with respect to the case where the projections are coated with the catalyst, it is needless to say that the same advantages can be achieved in the case where the wires illustrated in Example 1 or 2 are coated with the catalyst.

Example 5

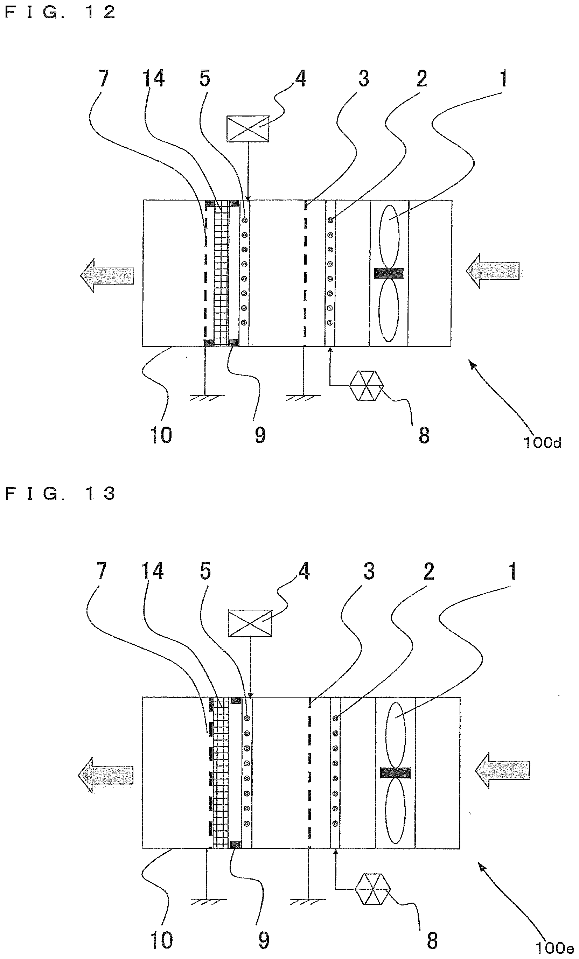

Fig. 12 is a sectional view illustrating a longitudinal section of a schematic configuration of an apparatus (hereinafter, referred to as the "apparatus 100d") for capture and inactivation of microbes and viruses according to Example 5. The configuration and operation of the apparatus 100d will be described with reference to Fig. 12. The difference between Example 5 and Examples 1 to 4 will be mainly described. The same components as those in Examples 1 to 4 are designated by the same reference numerals. The flow of air is indicated by arrows in Fig. 12.

While Examples 2 to 4 relate to modifications of the charging unit based on the structure in Example 1, Example 5 relates to a modification of the capturing unit based on the structure in Example 1. Specifically, while the capturing units in Examples 1 to 4 each include the capturing/inactivating-unit high-voltage electrode 5, the hydrophilic filter 6, and the capturing/inactivating-unit ground electrode 7 such that the capturing/inactivating-unit high-voltage electrode 5 is connected to the variable high voltage power supply 4, the capturing/inactivating-unit ground electrode 7 is grounded, and the hydrophilic filter 6 is sandwiched between the paired electrodes to capture airborne microorganisms, a hydrophilic filter in Embodiment 5 includes a honeycomb structure (hereinafter, referred to as the "honeycomb 14") supporting a hydrophilic absorbent on its surface as illustrated in Fig. 12.

The honeycomb 14 is configured such that the hydrophilic absorbent is supported on the surface of the honeycomb which comprises, for example, metal (e.g., stainless steel or aluminum), ceramic, or paper. Examples of the hydrophilic absorbent include hydrophilic zeolite, which is effective. Any absorbent exhibiting high hygroscopicity may be used. The honeycomb 14 can be formed by immersing, for example, a metal honeycomb member into a slurry solution containing activated carbon, drying the resultant member, and then firing the member at a proper temperature.

This configuration offers the advantages described in Embodiment 1 and further prevents airborne microorganisms charged by the charging unit from being formed into droplets on the surface of the honeycomb 14 upon collision with the honeycomb 14 subjected to electrostatic induction. Furthermore, the airborne microorganisms subjected to collision can be trapped in pores on the surface of the absorbent. Accordingly, an electric field generated around the honeycomb 14 prevents viruses and microbes from being scattered again and the viruses and microbes can be captured with high efficiency and be held as captured. Since the hydrophilic absorbent is used, odor components can also be captured.

As described above, the capturing unit including the capturing/inactivating-unit high-voltage electrode 5, the honeycomb 14, and the capturing/inactivating-unit ground electrode 7 achieves the advantage of capturing not only airborne microorganisms but also chemical substances, such as odor components, with high efficiency.

While Example 5 has been described with respect to the case where the honeycomb member made of, for example, metal is coated with the hydrophilic absorbent, a catalyzing substance, such as manganese dioxide ($MnO_2$), titanium dioxide ($TiO_2$), zinc oxide ($ZnO$), platinum (Pt), copper (Cu), or silver (Ag), may be supported on the absorbent. This configuration enables the catalyst to be activated upon plasma processing in the process of inactivating viruses and microbes with plasma or to convert discharge products into substances exhibiting higher activity. Consequently, viruses and microbes can be inactivated in a shorter time. Furthermore, chemical substances deposited on the honeycomb 14 can be decomposed and removed.

The honeycomb 14 may include at least two honeycombs (e.g., a hydrophilic honeycomb 14a and a catalyst-coated honeycomb 14b) as illustrated in Fig. 21. In this case, preferably, the hydrophilic honeycomb 14a is placed close to the charging unit (on the upstream side) and the catalyst-coated honeycomb 14b is placed far away from the charging unit (on the downstream side). In other words, it is only required that the honeycomb positioned closest to the charging unit is hydrophilic. Any other honeycombs should not be particularly limited. The catalyst-coated honeycomb 14b is coated with, for example, an absorbent for absorbing an odor gas or a catalyst for decomposing and reducing the above-described odor components. Note that the catalyst-coated honeycomb 14b may be hydrophilic or hydrophobic in this configuration. Preferably, the catalyst-coated honeycomb 14b includes a hydrophilic absorbent and a hydrophobic absorbent in combination, because the number of gases which can be absorbed or decomposed is increased.

In addition, since the honeycomb 14 enables the capturing unit to decompose discharge products (e.g., ozone) generated in the charging unit while capturing airborne microorganisms, the efficiency of charging airborne microorganisms by the charging unit can be increased. The apparatus 100d therefore maximizes the efficiency of capturing airborne microorganisms by the capturing unit and further increases the efficiency of removing viruses and microbes.

Example 6

Fig. 13 is a sectional view illustrating a longitudinal section of a schematic configuration of an apparatus (hereinafter, referred to as the "apparatus 100e") for capture and inactivation of microbes and viruses according to 6. The configuration and operation of the apparatus 100e will be described with reference to Fig. 13. The difference between Example 6 and Examples 1 to 5 will be mainly described. The same components as those in Examples 1 to 5 are designated by the same reference numerals. The flow of air is indicated by arrows in Fig. 13.

While Example 5 illustrates the case where the honeycomb 14 is disposed between the capturing/inactivating-unit high-voltage electrode 5 and the capturing/inactivating-unit ground electrode 7 such that the honeycomb 14 is not in contact with the electrodes, Example 6 relates to the configuration in which the honeycomb 14 is disposed in contact with the capturing/inactivating-unit ground electrode 7. Specifically, the configuration according to Example 6 is fundamentally similar to that according to Example 5 but Example 6 differs from Example 5 in that the honeycomb 14 is in contact with the capturing/inactivating-unit ground electrode 7. In this configuration, since the honeycomb 14 usually absorbs water, the surface resistance of the honeycomb 14 is reduced and the honeycomb 14 therefore becomes an electrical conductor. Accordingly, the honeycomb 14 is also grounded. In the capturing unit, therefore, an electric field is generated between the capturing/inactivating-unit high-voltage electrode 5 and the honeycomb 14.

In this configuration, since the honeycomb 14 is not subjected to electrostatic induction but the electric field is generated around the honeycomb 14, airborne microorganisms charged by the charging unit can be attracted by the electric field. Like the apparatus 100d according to Example 5, therefore, the apparatus 100e offers the advantages of capturing and inactivating viruses and microbes.

Note that viruses and microbes tend to be attracted to the honeycomb 14 on conditions that when the viruses and microbes are negatively charged by the charging unit, the polarity of a voltage applied to the high-voltage electrode for capture is set to negative, and when the viruses and microbes are positively charged by the charging unit, the polarity of a voltage applied to the high-voltage electrode for capture is set to positive. Thus, the viruses and microbes can be more efficiently captured. The same applies to the hydrophilic filter 6, in addition to the honeycomb 14.

The apparatus 100e according to Example 6 further has a unique advantage of relatively easily generating plasma between the capturing/inactivating-unit high-voltage electrode 5 and the honeycomb 14 by setting a voltage applied from the variable high voltage power supply 4 to the capturing/inactivating-unit high-voltage electrode 5 to be higher than that upon capturing viruses and microbes. This is because the discharge distance can be reduced by the thickness of the honeycomb 14. In the process of inactivating viruses and microbes with plasma, therefore, viruses and microbes captured on the honeycomb 14 can be inactivated with high efficiency by plasma between the capturing/inactivating-unit high-voltage electrode 5 and the honeycomb 14.

Example 6A

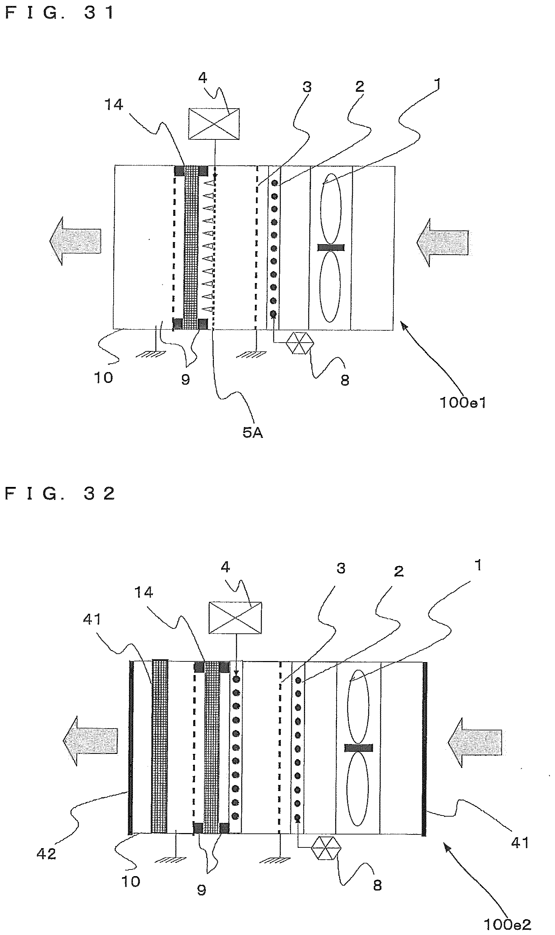

Fig. 31 is a sectional view illustrating a longitudinal section of a schematic configuration of an apparatus (hereinafter, referred to as the "apparatus 100e1 ") for capture and inactivation of microbes and viruses according to Example 6A. The configuration and operation of the apparatus 100e1 will be described with reference to Fig. 31. The difference between Example 6A and Examples 1 to 6 will be mainly described. The same components as those in Examples 1 to 6 are designated by the same reference numerals. The flow of air is indicated by arrows in Fig. 31.

Examples 2 to 4 relate to the modifications of the charging unit based on the configuration in Example 1 and Examples 5 and 6 relate to modifications of the capturing unit, specially, the hydrophilic filter. Furthermore, Example 6A relates to a modification of the virus inactivating unit based on the configuration in Example 1. Note that the capturing/inactivating-unit high-voltage electrode 5, the hydrophilic filter 6 (or the honeycomb 14), and the capturing/inactivating-unit ground electrode 7 constitute the virus inactivating unit.

In the virus inactivating unit in Example 1, the capturing/inactivating-unit high-voltage electrode 5 including wires, the hydrophilic filter 6, and the capturing/inactivating-unit ground electrode 7 including metal mesh are arranged in that order from the windward side to charge airborne microorganisms. In Example 6A, an electrode (hereinafter, referred to as the "capturing/inactivating-unit high-voltage electrode 5A") including a plurality of projections is included instead of the capturing/inactivating-unit high-voltage electrode 5. For example, the projections may be attached to metal mesh or a plate, which permits the air to pass therethrough without pressure loss, by welding or the like to constitute the capturing/inactivating-unit high-voltage electrode 5A. According to another modification, a metal plate may be cut with a wire cutter or the like to form projections, thus constituting the capturing/inactivating-unit high-voltage electrode 5A.

This configuration offers the advantages described in Example 1 and further prevents the capturing/inactivating-unit high-voltage electrode 5A from being damaged due to abnormal discharge caused by, for example, dust entering from the outside, so that stable discharge can be easily maintained. In addition, discharge by plasma easily occurs.

Example 6B

Fig. 32 is a sectional view illustrating a longitudinal section of a schematic configuration of an apparatus (hereinafter, referred to as the "apparatus 100e2") for capture and inactivation of microbes and viruses according to Example 6B. The configuration and operation of the apparatus 100e2 will be described with reference to Fig. 32. The difference between Example 6B and Examples 1 to 6 and 6A will be mainly described. The same components as those in Examples 1 to 6 and 6A are designated by the same reference numerals. The flow of air is indicated by arrows in Fig. 32.

The apparatus 100e2 is configured such that the apparatus includes an ozone decomposition catalytic filter 41 and air-path opening and closing devices (an inlet opening and closing device 42 and an outlet opening and closing device 43) in addition to the components of the apparatus 100 according to Example 1. The ozone decomposition catalytic filter 41 is placed downstream of the hydrophilic filter 6 (or the honeycomb 14). The opening and closing devices are arranged on a virus inlet and a virus outlet (an air inlet and an air outlet) of the apparatus 100e2, respectively. Specifically, the inlet opening and closing device 42 is positioned at the air inlet and the outlet opening and closing device 43 is positioned at the air outlet.

The ozone decomposition catalytic filter 41 may be any filter having a function of ozone decomposition. For example, in the use of an ozone decomposition catalyst, such as activated carbon, the ozone decomposition catalytic filter 41 can be formed by immersing a filter member into a slurry solution containing the catalyst used, drying the resultant member, and then firing the member at a proper temperature.

The opening and closing devices may be of any type capable of preventing the outflow of an ozone gas generated after closing the air path. For example, attaching remote-controllable or automatically closable plastic plates to the air inlet and the air output, respectively, enables the functions of the opening and closing devices. If the attached plates are coated with an ozone decomposition catalyst, the risk of outflow of an ozone gas generated in the air path from the apparatus will be further reduced. It is therefore more effective.

The operation of the apparatus 100e2 will now be described.

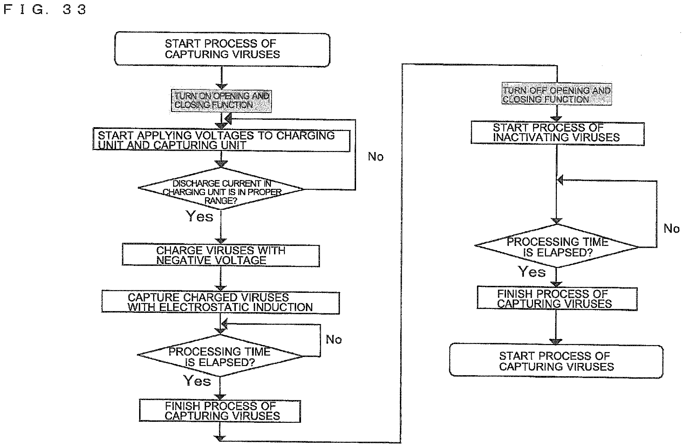

Fig. 33 is a flowchart illustrating the flow of a method for capture and inactivation of microbes and viruses, the method being executed by the apparatus 100e2. The fundamental operation is as described in Example 1 with reference to Fig. 3. The difference between this method and that according to Example 1, namely, a feature of Example 6B is that a positive voltage is applied in the process of capturing microbes and viruses while the opening and closing devices are opened and a negative voltage is applied in the process of inactivating the captured microbes and viruses while the opening and closing devices are closed. Consequently, the microbes and viruses can be efficiently removed.

As illustrated in Fig. 8, there is a correlation between the survival rate and the product (ppm·min) of the concentration of ozone gas and time in processing with plasma. Accordingly, in the case where viruses are intended to be inactivated to a certain survival rate, if the concentration of ozone gas is increased, the processing time becomes shorter.

This configuration offers the advantages described in Example 1 and further enables efficient capture of only microbes and viruses without generating an ozone gas in the process of capturing microbes and viruses. Moreover, this configuration enables, in the process of inactivating the captured microbes and viruses, efficient ozone gas generation and an increase in concentration of the ozone gas while preventing the ozone gas from flowing out from the apparatus. Advantageously, the viruses captured by the filter can be efficiently inactivated in a short time. Furthermore, since the ozone decomposition catalyst is capable of capturing and decomposing odor components, the odor components can also be captured.

The ozone decomposition catalytic filter 41 may have a honeycomb structure. Furthermore, if the hydrophilic filter is coated with a decomposition catalyst, the same advantage can be achieved without the filter. In addition, if air path portions outside the electrodes are extended, the same advantage as that offered by the opening and closing devices can be achieved.

As regards the concentration of ozone gas, the gas spreads by diffusion. Accordingly, in the case where the concentration of ozone gas is low, alternatively, the processing time is short, the same advantage can be achieved by merely placing the opening and closing device at the outlet of the air path. The following opening and closing device may be disposed as such an air-path opening and closing device.

Moving air has a force. The air contains nitrogen and oxygen at 1 atmospheric pressure and has a mass of approximately 1.3 kg/m$^3$. Assuming that the quantity of air sent is 1.0 m/s in the process of capturing viruses and the air path has a diameter of $\phi$10cm, 1 momentum applied to the opening and closing device is estimated to be approximately 1 g /opening and closing device by the following Expression 1. Accordingly, if the opening and closing device has a mass of approximately 1 g, the opening and closing device can be configured such that the air path is opened due to the air sent in the process of capturing viruses and the air path is closed during virus inactivation.

$$\Delta P = m*V \qquad \qquad \text{… Expression 1}$$

where m = the mass (kgW) of air that collides with the device per second = the product (1.3 kg/m$^3$) of the volume of air that collides with the device per second and the density of air, and
V = air velocity (m/s)

Example 6C

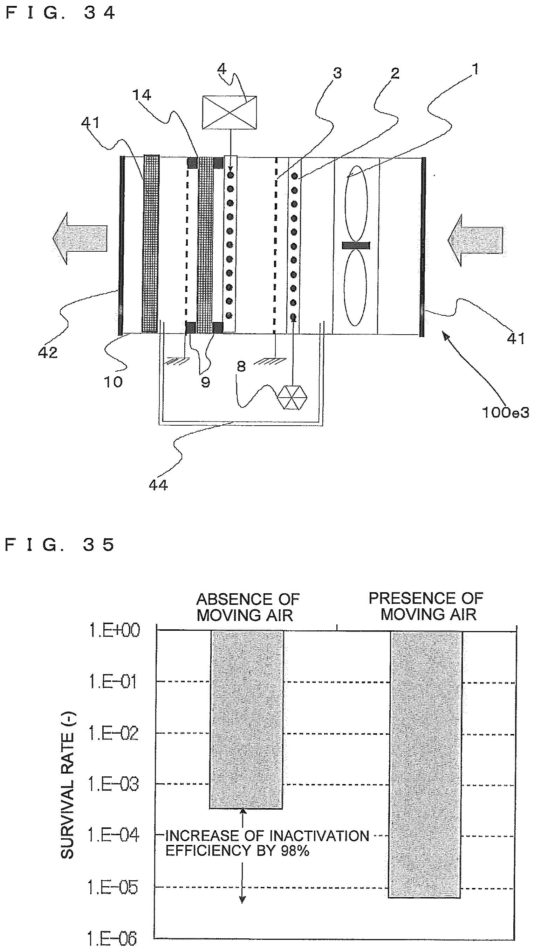

Fig. 34 is a sectional view illustrating a longitudinal section of a schematic configuration of an apparatus (hereinafter, referred to as the "apparatus 100e3") for capture and inactivation of microbes and viruses according to Example 6C. The configuration and operation of the apparatus 100e3 will be described with reference to Fig. 34. The difference between Example 6C and Examples 1 to 6, 6A, and 6B will be mainly described. The same components as those in Examples 1 to 6, 6A, and 6B are designated by the same reference numerals. The flow of air is indicated by arrows in Fig. 34.

The apparatus 100e3 is configured such that the apparatus includes a bypass 44 in addition to the components of the apparatus 100 according to Example 1. One end of the bypass 44 is connected to the downstream side of the air-sending device 1 (or the upstream side of the charging-unit high-voltage electrode 2) and the other end thereof is connected to the upstream side of the ozone decomposition catalytic filter 41 in the air path housing 10. The bypass 44 is configured to circulate the air in the air path housing 10. The bypass 44 can be made of any insulating material.

Fig. 35 is a graph of the effect of moving air on virus inactivation with plasma. In Fig. 35, the axis of abscissas indicates the presence or absence of moving air and the axis of ordinates indicates the virus survival rate.

As illustrated in Fig. 35, the presence of moving air upon virus inactivation increased the efficiency of inactivation by 98%. This fact indicates that the presence of moving air upon virus inactivation increases the efficiency of virus inactivation. Accordingly, the apparatus 100e3 offers the advantages described in Embodiments 1 to 6 and 6A and further enables efficient capture of only microbes and viruses without generating an ozone gas in the process of capturing microbes and viruses. Moreover, the apparatus 100e3 enables, in the process of inactivating captured microbes and viruses, efficient ozone gas generation and ozone gas circulation by moving air while preventing the ozone gas from flowing out from the air path housing 10 and increasing the concentration of the gas. Advantageously, viruses captured by the filter can be more efficiently inactivated in a shorter time.

Example 7

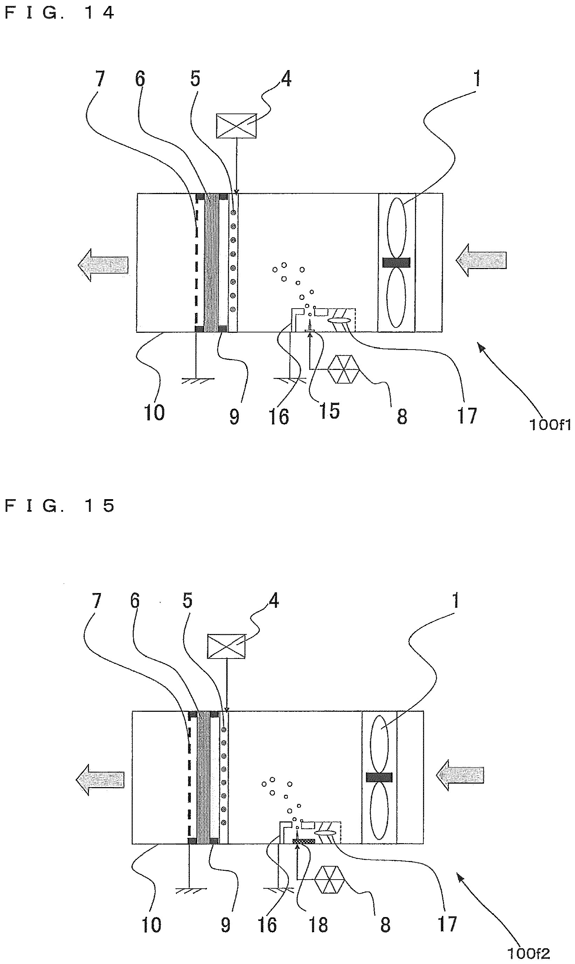

Fig. 14 is a sectional view illustrating a longitudinal section of a schematic configuration of an apparatus (hereinafter, referred to as the "apparatus 100f1") for capture and inactivation of microbes and viruses according to Examples 7. Fig. 15 is a sectional view illustrating a longitudinal section of a schematic configuration of an apparatus (hereinafter, referred to as the "apparatus 100f2") for capture and inactivation of microbes and viruses according to a modification of Example 7. The configurations and operations of the apparatuses 100f1 and 100f2 will be described with reference to Figs. 14 and 15. The difference between Example 7 and Examples 1 to 6 will be mainly described. The same components as those in Examples 1 to 6 (including Embodiments 6A to 6C, the same shall apply hereinafter) are designated by the same reference numerals. The flow of air is indicated by arrows in Figs. 14 and 15.

Examples 1 to 6 relate to the configurations in which the charging-unit high-voltage electrode (the charging-unit high-voltage electrode 2, the charging-unit high-voltage electrode 12, or the charging-unit high-voltage electrode 13) is disposed on the windward side and the charging-unit ground electrode (the charging-unit ground electrode 3 or the charging-unit ground electrode 11) is disposed on the leeward side to charge airborne microorganisms. According to Examples 7, as illustrated in Fig. 14, an ion generating unit (corresponding to the charging unit) including a discharge electrode (first high-voltage application electrode) 15, a ground electrode (first ground electrode) 16, a fan 17, and the high voltage power supply 8 is disposed on, for example, an inner wall of the air path housing 10 to charge airborne microorganisms with ions generated.

As illustrated in Fig. 15, the apparatus 100f2 is configured such that a charged-mist generating unit (corresponding to the charging unit) including a charged-mist spray electrode (first high-voltage application electrode) 18, the ground electrode 16, the fan 17, and the high voltage power supply 8 is disposed on, for example, an inner wall of the air path housing 10. Airborne microorganisms may be charged with charged mist.

The configurations of the apparatuses 100f1 and 100f2 enable the charging unit to have a compact configuration, though the number of components is increased.

Embodiment 1

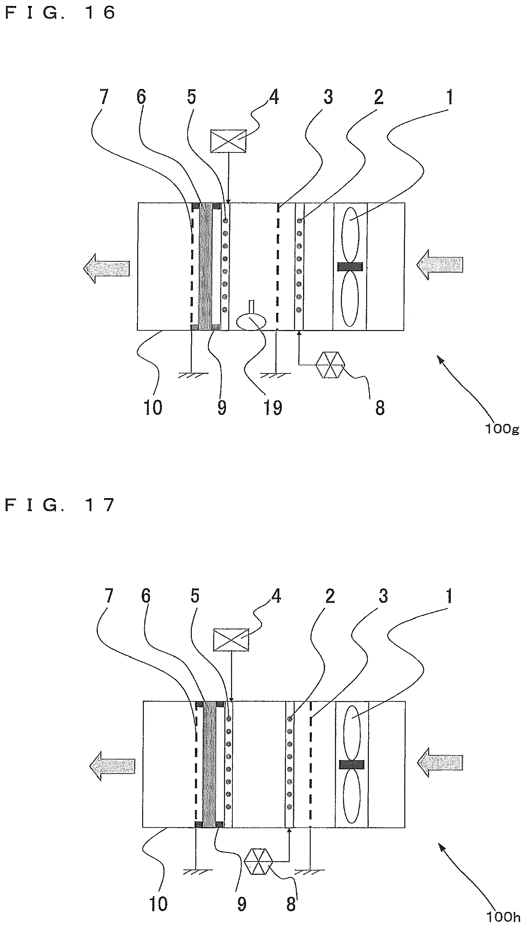

Fig. 16 is a sectional view illustrating a longitudinal section of a schematic configuration of an apparatus (hereinafter, referred to as the "apparatus 100g") for capture and inactivation of microbes and viruses according to Embodiment 1 of the present invention. The configuration and operation of the apparatus 100g will be described with reference to Fig. 16. The difference between Embodiment 1 and Examples 1 to 7 will be mainly described. The same components as those in Examples 1 to 7 are designated by the same reference numerals. The flow of air is indicated by arrows in Fig. 16.

Examples 1 to 6 relate to the configurations in which the charging-unit high-voltage electrode (the charging-unit high-voltage electrode 2, the charging-unit high-voltage electrode 12, or the charging-unit high-voltage electrode 13) is disposed on the windward side and the charging-unit ground electrode (the charging-unit ground electrode 3 or the charging-unit ground electrode 11) is disposed on the leeward side to charge airborne microorganisms. According to Embodiment 8, as illustrated in Fig. 16, a humidifier 19 is disposed downwind of the charging unit including the charging-unit high-voltage electrode 2 and the charging-unit ground electrode 3 to mix airborne microorganisms, charged by the charging unit, with water supplied from the humidifier 19.

This configuration offers the advantages described in Examples 1 to 6 and further enables charged airborne microorganisms to be supplied with moisture.

Advantageously, the advantage of capturing airborne microorganisms through the capturing unit can be further increased.

While Embodiment 1 illustrates the case where the humidifier 19 is positioned downwind of the charging unit including the charging-unit high-voltage electrode 2 and the charging-unit ground electrode 3, in an Example outside the scope of the invention the humidifier 19 may be placed upwind of the charging unit including the charging-unit high-voltage electrode 2 and the charging-unit ground electrode 3. In this configuration, the charging-unit high-voltage electrode 2 has to be properly insulated because air containing water vapor is supplied to the charging-unit high-voltage electrode 2, though water containing airborne microorganisms is efficiently charged.

Example 8

Fig. 17 is a sectional view illustrating a longitudinal section of a schematic configuration of an apparatus (hereinafter, referred to as the "apparatus 100h") for capture and inactivation of microbes and viruses according to Example 8. The configuration and operation of the apparatus 100h will be described with reference to Fig. 17. The difference between Example 8 and Examples 1 to 7 and Embodiment 1 will be mainly described. The same components as those in Examples 1 to 7 and Embodiment 1 are designated by the same reference numerals. The flow of air is indicated by arrows in Fig. 17.

Examples 1 to 6 relate to the configurations in which the charging-unit high-voltage electrode (the charging-unit high-voltage electrode 2, the charging-unit high-voltage electrode 12, or the charging-unit high-voltage electrode 13) is disposed on the windward side and the charging-unit ground electrode (the charging-unit ground electrode 3 or the charging-unit ground electrode 11) is disposed on the leeward side to charge airborne microorganisms. According to Example 8, as illustrated in Fig. 17, the charging-unit high-voltage electrode 2 is disposed on the leeward side and the charging-unit ground electrode 3 is disposed on the windward side to charge airborne microorganisms.

This configuration enables an electric field generated by the charging-unit high-voltage electrode 2 and the capturing/inactivating-unit high-voltage electrode 5 to be stronger than that generated by the charging-unit ground electrode 3 and the capturing/inactivating-unit high-voltage electrode 5, such that airborne microorganisms tend to be attracted to the capturing/inactivating-unit high-voltage electrode 5. Specifically, the apparatus 100h, configured such that the charging-unit high-voltage electrode 2 is disposed on the leeward side and the charging-unit ground electrode 3 is disposed on the windward side, has the advantage of removing airborne microorganisms with higher efficiency.

Example 9

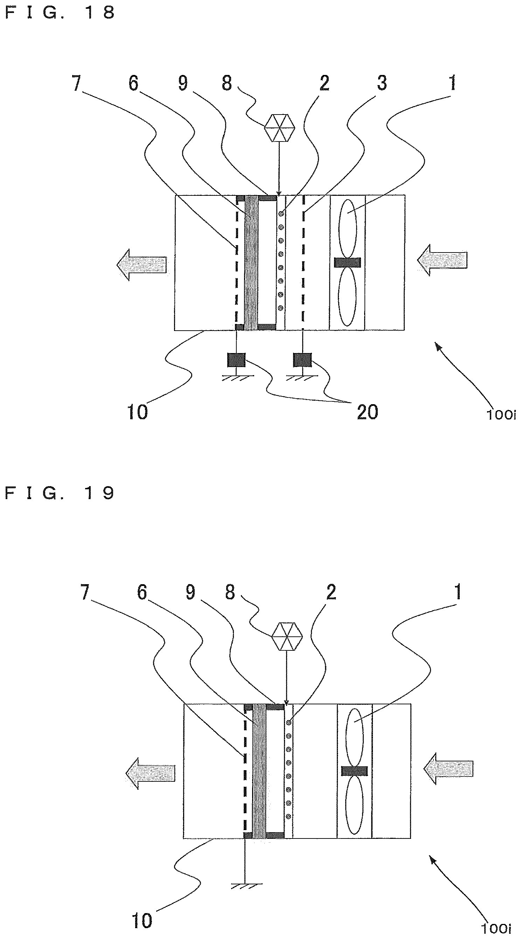

Fig. 18 is a sectional view illustrating a longitudinal section of a schematic configuration of an apparatus (hereinafter, referred to as the "apparatus 100i") for capture and inactivation of microbes and viruses according to Example 9. The configuration and operation of the apparatus 100i will be described with reference to Fig. 18. The difference between Example 9 and Examples 1 to 8 and Embodiment 1 will be mainly described. The same components as those in Examples 1 to 8 and Embodiment 1 are designated by the same reference numerals. The flow of air is indicated by arrows in Fig. 18.

While Example 8 relates to the configuration in which the charging-unit high-voltage electrode 2 is disposed on the leeward side and the charging-unit ground electrode 3 is disposed on the windward side to charge airborne microorganisms, Embodiment 9 relates to the configuration in which the capturing/inactivating-unit high-voltage electrode 5 and the variable high voltage power supply 4 are detached and the charging-unit high-voltage electrode 2 disposed on the leeward side functions as the capturing/inactivating-unit high-voltage electrode 5 as illustrated in Fig. 18.

In this configuration, a power switch 20 is provided for each of the charging-unit ground electrode 3 and the capturing/inactivating-unit ground electrode 7 in order to enable switching between discharge spaces. In the process of charging and capturing airborne viruses and airborne microbes, the power switch 20 connected to the charging-unit ground electrode 3 may be turned on to ground the electrode. In the process of inactivating captured viruses and microbes, the power switch 20 connected to the capturing/inactivating-unit ground electrode 7 may be turned on to ground the electrode.

This configuration enables a reduction in the number of components constituting the apparatus 100i, so that the apparatus 100i can be provided at low cost. Specifically, since the apparatus 100i includes the charging-unit ground electrode 3, the charging-unit high-voltage electrode 2, the high voltage power supply 8, the hydrophilic filter 6, the capturing/inactivating-unit ground electrode 7, the bushings 9, and the power switches 20, the apparatus 100i can be made at lower cost. Since discharge conditions for charging airborne microorganisms hardly agree with those for inactivating captured viruses and microbes, however, the advantage of removing airborne microorganisms is lower than those described in Embodiments 1 to 9.


Example 10

Fig. 19 is a sectional view illustrating a longitudinal section of a schematic configuration of an apparatus (hereinafter, referred to as the "apparatus 100j") for capture and inactivation of microbes and viruses according to Example 10. The configuration and operation of the apparatus 100j will be described with reference to Fig. 19. The difference between Example 10 and Examples 1 to 9 and Embodiment 1 will be mainly described. The same components as those in Examples 1 to 9 and Embodiment 1 are designated by the same reference numerals. The flow of air is indicated by arrows in Fig. 19.

While Example 9 relates to the configuration in which the apparatus 100i includes the charging-unit ground electrode 3, the charging-unit high-voltage electrode 2, the high voltage power supply 8, the hydrophilic filter 6, the capturing/inactivating-unit ground electrode 7, the bushings 9, and the power switches 20, Example 10 relates to the configuration in which the apparatus 100j includes the charging-unit high-voltage electrode 2, the high voltage power supply 8, the hydrophilic filter 6, the capturing/inactivating-unit ground electrode 7, and the bushings 9 as illustrated in Fig. 19.

In this configuration, the process of capturing viruses and microbes and the process of inactivating viruses and microbes are simultaneously performed. Specifically, airborne microorganisms ionized by, for example, electrons and ions are captured by the hydrophilic filter 6 and the captured airborne microorganisms are supplied with discharge products (e.g., ozone and radicals) generated by discharge, so that the captured viruses and microbes are inactivated.

This configuration enables a reduction in the number of components constituting the apparatus 100j, so that the apparatus 100j can be provided at low cost. In other words, since the apparatus 100j includes the charging-unit high-voltage electrode 2, the high voltage power supply 8, the hydrophilic filter 6, the capturing/inactivating-unit ground electrode 7, and the bushings 9, the apparatus 100j can be made at lower cost. Since discharge conditions for charging airborne viruses and airborne microbes hardly agree with those for inactivating captured viruses and microbes, however, the advantage of removing airborne microbes and airborne viruses is lower than those described in Examples 1 to 8 and Embodiment 1.


Example 11

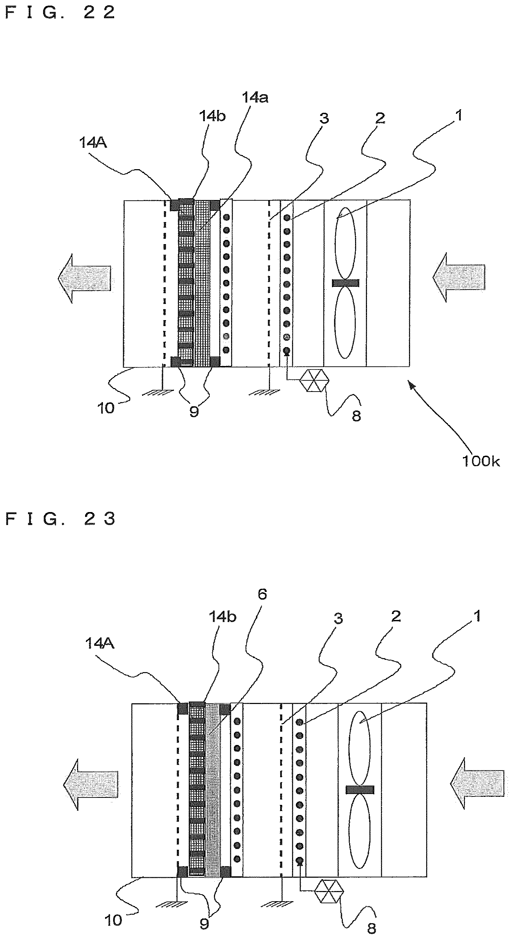

Fig. 22 is a sectional view illustrating a longitudinal section of a schematic configuration of an apparatus (hereinafter, referred to as the "apparatus 100k") for capture and inactivation of microbes and viruses according to Example 12. The configuration and operation of the apparatus 100k will be described with reference to Fig. 22. The difference between Example 11 and Examples 1 to 10 and Embodiment 1 will be mainly described. The same components as those in Examples 1 to 10 and Embodiment 1 are designated by the same reference numerals. The flow of air is indicated by arrows in Fig. 22. Furthermore, in Example 12, the honeycomb 14 includes at least two kinds of honeycombs of Example 5 as illustrated in Fig. 21.

Example 11 illustrates the case where the honeycombs 14 are sandwiched between the bushings 9 such that the honeycombs 14 are not in contact with the electrodes. If the bushing 9 is an insulator having a honeycomb structure (a honeycomb 14A illustrated in Fig. 22), the same configuration can be achieved. Furthermore, the honeycomb structure may be coated with an insulating oxide catalyst (e.g., titanium oxide, manganese oxide, zirconium oxide, or copper oxide). The hydrophilic honeycomb 14a at the first stage captures and inactivates microbes and viruses and the catalyst-coated honeycomb 14b on the second stage decomposes ozone generated by discharge, and radical particles generated upon decomposition can remove smell and decompose a harmful gas. While the case where the hydrophilic honeycomb serves as the first-stage filter has been described, the use of the hydrophilic filter 6, as illustrated in Fig. 23, instead of the hydrophilic honeycomb 14a offers the same advantages.

Example 12

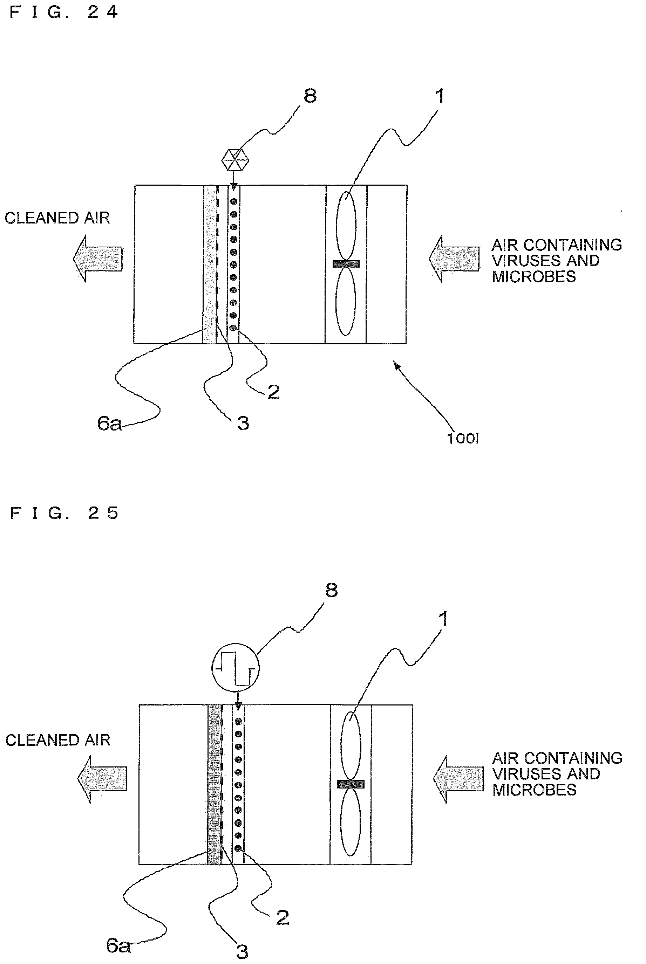

Fig. 24 is a sectional view illustrating a longitudinal section of a schematic configuration of an apparatus (hereinafter, referred to as the "apparatus 100l") for capture and inactivation of microbes and viruses according to Example 12. The difference between Example 12 and Examples 1 to 11 and Embodiment 1 will be mainly described. The same components as those in Examples 1 to 11 and Embodiment 1 are designated by the same reference numerals. The flow of air is indicated by arrows in Fig. 24.

While Example 9 illustrates the case in which the hydrophilic filter 6 is placed so as to be sandwiched between the charging-unit high-voltage electrode 2 and the capturing/inactivating-unit ground electrode 7, Example 12 illustrates a case in which a hydrophilic filter 6a previously charged is placed on the opposite side of the ground electrode from the high-voltage electrode. Viruses charged by the charging unit are captured on the hydrophilic filter polarized by the electrostatic field and are then processed in Example 9, whereas the apparatus 1001 enables charged viruses to be captured by the hydrophilic filter 6a without being polarized by the electrostatic field, because the filter has been previously charged.

The placement of the previously charged hydrophilic filter 6a in the vicinity of the ground electrode enables captured viruses to collide with radicals and ozone generated by discharge between the charging-unit high-voltage electrode 2 and the charging-unit ground electrode 3 before the radicals and ozone collide with other particles and then disappear. Accordingly, the apparatus 1001 offers a more effective advantage of inactivating microbes and viruses. Furthermore, as illustrated in Fig. 25, if the voltage applied to the charging-unit high-voltage electrode 2 is alternating or has a positive-negative alternating rectangular waveform or a positive-negative alternating pulsed waveform, or, if a DC positive voltage and a DC high voltage are alternatively applied to the charging-unit high-voltage electrode 2, charged viruses captured and inactivated by the hydrophilic filter 6a can be allowed to collide with particles with charges of opposite polarity, so that charges can be neutralized and the resultant charges can be released into a space. This prevents deposition of particles on the hydrophilic filter 6a, such that the filter can be kept clean for a long period.

Example 12A

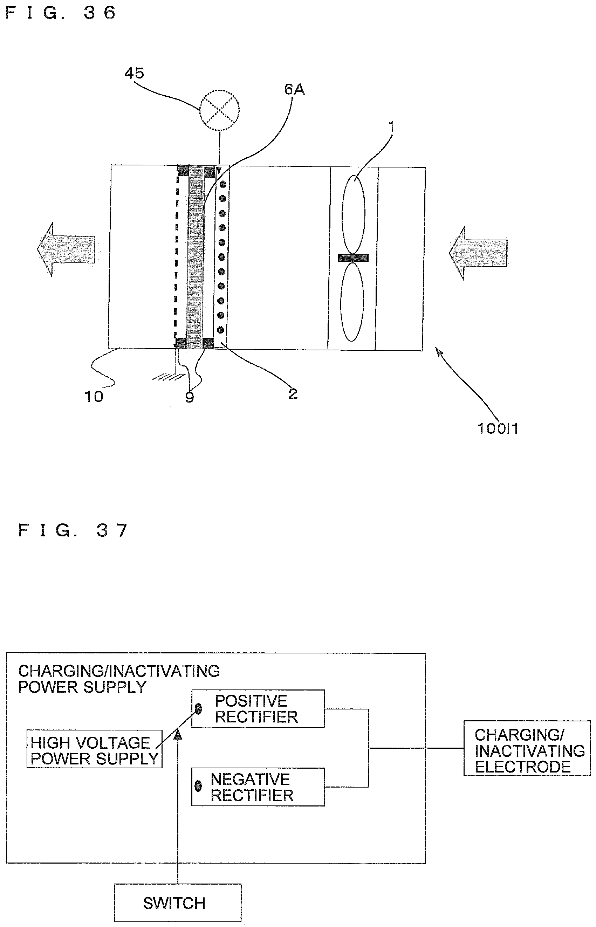

Fig. 36 is a sectional view illustrating a longitudinal section of a schematic configuration of an apparatus (hereinafter, referred to as the "apparatus 100l1") for capture and inactivation of microbes and viruses according to Example 12A. The difference between Example 13A and Examples 1 to 11 and Embodiment 1 will be mainly described. The same components as those in Embodiments 1 to 12 and embodiment 1 are designated by the same reference numerals. The flow of air is indicated by arrows in Fig. 36.

While Example 12uses the previously charged hydrophilic filter 6a such that the filter is placed in the vicinity of the ground electrode, Example 12A uses the previously charged hydrophilic filter 6a such that the filter is placed in the vicinity of the ground electrode and is separated from the charging-unit high-voltage electrode 2 by a distance of 20 mm or less. The charging-unit high-voltage electrode 2 is connected to a charging/inactivating high voltage power supply 45.

Furthermore, as illustrated in Fig. 37, the charging/inactivating high voltage power supply 45 is a power supply configured such that a high voltage power supply can be switched between connection to a positive rectifier and connection to a negative rectifier. In the charging/inactivating high voltage power supply 45, the use of alternating current, a positive-negative alternating rectangular waveform, a positive-negative alternating pulsed waveform, or the like in the high voltage power supply enables flexible voltage application on the charging-unit high-voltage electrode 2 using the rectifier connected at the following stage while switching between the positive high voltage and the negative high voltage.

Consequently, in Example 12A, the charging/inactivating high voltage power supply 45 applies the positive high voltage to the charging-unit high-voltage electrode 2 in the process of capturing viruses, while the charging/inactivating high voltage power supply 45 applies the negative high voltage to the charging-unit high-voltage electrode 2 upon virus inactivation. This configuration enables a reduction in the number of components constituting the apparatus 100l1, so that the apparatus 100l1 can be provided at low cost.

**EP 2 578 243 B1**

Specifically, in the process of capturing microbes and viruses, only microbes and viruses can be efficiently captured using the single combination of the charging/inactivating high voltage power supply 45 and the charging-unit high-voltage electrode 2 without ozone gas generation, similar to the advantages described in Example 1 to 12 and Embodiment 1. Furthermore, in the process of inactivating captured microbes and viruses, viruses captured on the filter can be efficiently inactivated in a short time while an ozone gas is being efficiently generated and the concentration of the gas is being raised. While the power supply as illustrated in Fig. 37 is used as the charging/inactivating high voltage power supply 45, any power supply capable of achieving periodic alternate application of a positive voltage and a negative voltage offers the same advantages as those of Embodiment 13A.

Example 13

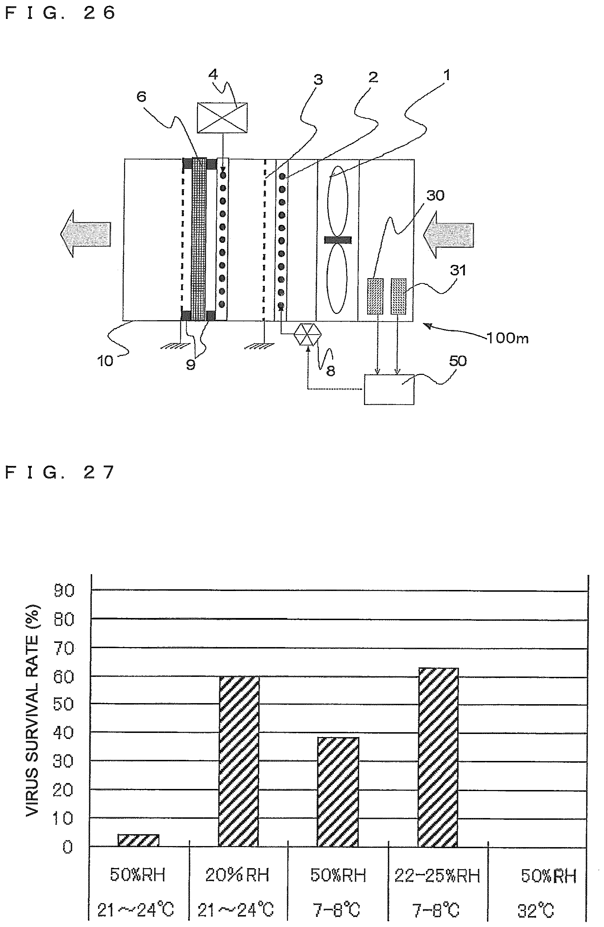

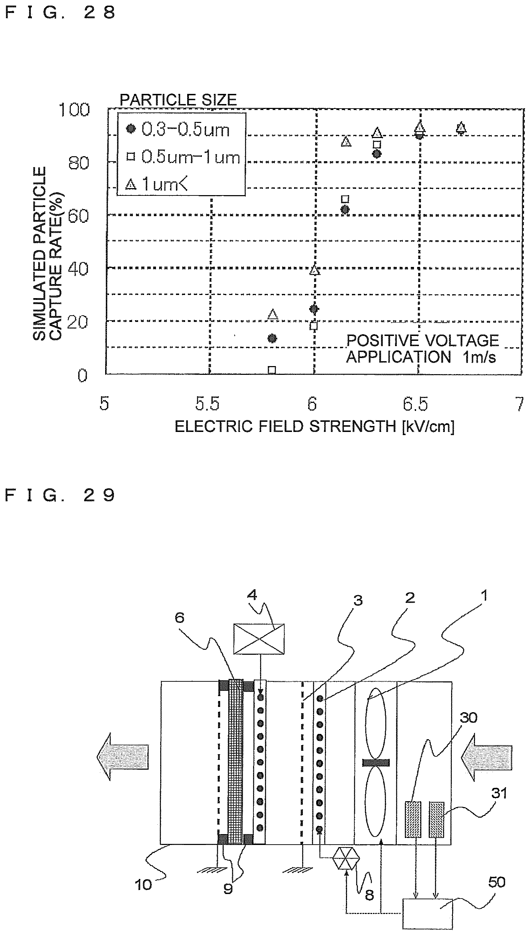

Fig. 26 is a sectional view illustrating a longitudinal section of a schematic configuration of an apparatus (hereinafter, referred to as the "apparatus 100m") for capture and inactivation of microbes and viruses according to Example 13. The difference between Example 13 and Examples 1 to 12 (including Example 12A, the same shall apply hereinafter) and Embodiment 1 will be mainly described. The same components as those in Examples 1 to 12 and Embodiment 1 are designated by the same reference numerals. The flow of air is indicated by arrows in Fig. 26.
The apparatus 100m according to Example 13 is configured such that the apparatus includes a temperature and humidity sensor 30, a dust sensor 31, and a controller 50 in addition to the components of the apparatus 100 according to Example 1. The temperature and humidity sensor 30 and the dust sensor 31 are arranged at a virus inlet (air inlet 10a). The controller 50 is configured to transmit an output signal indicating information obtained by the temperature and humidity sensor 30 and/or the dust sensor 31 to the high voltage power supply 8.
Typical viruses are suspended while being contained in moisture and are said to have sizes ranging from 0.3 to 0.5 $\mu$m, namely, at or below 1 $\mu$m. Furthermore, microbes, such as bacteria and mold, have a size of 1 $\mu$m. Fig. 27 illustrates a change in influenza virus survival rate with varying temperature and humidity after being left for six hours as it was. Fig. 27 demonstrates that the activity of the virus increased under low temperature and low humidity conditions, whereas it decreased under high temperature and high humidity conditions. Furthermore, it is known that the activity of microbes increases under relatively high temperature conditions and it decreases under low humidity conditions, because microbes are sensitive to drying.
As features of the present invention, the efficiency of capture varies depending on particle size. Fig. 28 is a graph illustrating features of the rate of particle capture depending on particle size. In Fig. 28, the axis of abscissas indicates the strength (kV/cm) of the electric field between the charging-unit high-voltage electrode 2 and the charging-unit ground electrode 3 and the axis of ordinates indicates the particle capture rate (%). As illustrated in Fig. 28, the rates of capture of particles having diameters above 1 $\mu$m significantly differ from those at and below 1 $\mu$m. In particular, while the rate of capture of particles having diameters at and above 1 $\mu$m is 88% at an electric field strength of 6.15 kV/cm, the rate of capture of particles having diameters at and below 1 $\mu$m ranges from 60% to 66% at that electric field strength. The above facts indicate that unnecessary energy consumption can be eliminated by changing the strength of the electric field generated upon capture of viruses and microbes with different particle diameters.
Tables 2 to 4 illustrate the relationships of a processing mode and an output of the temperature and humidity sensor 30 and/or an output of the dust sensor 31. The processing modes of the apparatus 100m will be described on the basis of Tables 2 to 4.

[Table 2]

| Temperature | low | low | high | high |
|---|---|---|---|---|
| Humidity | low | high | low | high |
| Processing mode | virus | stop or virus | stop or microbe | microbe |

[Table 3]

| Temperature | low | | low | | high | | high | |
|---|---|---|---|---|---|---|---|---|
| Humidity | low | | high | | low | | high | |
| Dust | low | high | low | high | low | high | low | high |
| Processing mode | virus | virus | stop | virus | stop | microbe | microbe | microbe |

[Table 4]

| | | | | | | | | | | | | | | | | | |
|---|---|---|---|---|---|---|---|---|---|---|---|---|---|---|---|---|---|
| Temperature | | high | high | high | high | high | high | high | high | low | low | low | low | low | low | low | low |
| Humidity | | high | high | high | high | low | low | low | low | high | high | high | high | low | low | low | low |
| Dust | 1 μm ≤ | high | high | low | low | high | high | low | low | high | high | low | low | high | high | low | low |
| | 1 μm ≥ | high | low | high | low | high | low | high | low | high | low | high | low | high | low | high | low |
| Processing mode | | microbe | microbe | microbe | microbe | microbe | stop | microbe | stop | virus | virus | virus | stop | virus | virus | virus | virus |

Referring to Table 2, the apparatus 100m is configured to perform processes in the virus processing mode under low temperature conditions and perform processes in the microbe processing mode under high temperature conditions. In addition, as illustrated in Table 3, an output of the dust sensor 31 may be taken into consideration. When the amount of dust is high, the processes may be performed. When the amount of dust is low, the processes may be stopped. Furthermore, if the dust sensor 31 can determine the particle size of dust, the processes may be changed on the basis of whether the particle size is at or below 1 μm as illustrated in Table 4. Furthermore, as illustrated in Fig. 29, the operation of the air-sending device 1 may be controlled on the basis of an output signal from the controller 50 such that the air-sending device 1 is suspended while such a virus device is being stopped. Thus, unnecessary energy consumption can be further avoided.

While the apparatuses and methods for capture and inactivation of microbes and viruses according to the present invention have been described with respect to Examples 1 to 13 and Embodiment 1, the features of Examples and Embodiments may be properly combined to provide an apparatus and method for capture and inactivation of microbes and viruses.

Reference Signs List

1, air-sending device; 2, charging-unit high-voltage electrode; 3, charging-unit ground electrode; 4, variable high voltage power supply; 5, capturing/inactivating-unit high-voltage electrode; 5A, capturing/inactivating-unit high-voltage electrode; 6, hydrophilic filter; 6a, hydrophilic filter; 7, capturing/inactivating-unit ground electrode; 8, high voltage power supply; 9, bushing; 10, air path housing; 10a, air inlet; 11, charging-unit ground electrode; 12, charging-unit high-voltage electrode; 13, charging-unit high-voltage electrode; 14, honeycomb; 14A, honeycomb; 14a, hydrophilic honeycomb; 14b, catalyst-coated honeycomb; 16, ground electrode; 17, fan; 19, humidifier; 20, power switch; 30, temperature and humidity sensor; 31, dust sensor; 41, ozone decomposition catalytic filter; 42, inlet opening and closing device; 43, outlet opening and closing device; 44, bypass; 45, charging/inactivating high voltage power supply; 50, controller; 100, apparatus; 100a, apparatus; 100b, apparatus; 100c, apparatus; 100d, apparatus; 100e, apparatus; 100e1, apparatus; 100e2, apparatus; 100e3, apparatus; 100f1, apparatus; 100f2, apparatus; 100g, apparatus; 100h, apparatus; 100i, apparatus; 100j, apparatus; 100k, apparatus; 100l, apparatus; 100ll, apparatus; and 100m, apparatus.

**Claims**

1. An apparatus (100) for capture and inactivation of microbes and viruses, comprising:

    an air path housing (10);
    a first electrode (2) configured to be applied with a voltage to charge airborne microorganisms introduced in the air path housing (10);
    a first counter electrode (3) placed so as to face the first electrode (2);
    a high voltage power supply (8) configured to apply a voltage between the first electrode (2) and the first counter electrode (3);
    a hydrophilic filter (6) that captures the airborne microorganisms charged by discharge generated between the first electrode (2) and the first counter electrode (3);
    a second electrode (5) configured to subject the hydrophilic filter (6) to electrostatic induction;
    a second counter electrode (7) placed so as to face the second electrode (5) with the hydrophilic filter (6) being sandwiched therebetween;
    a high voltage power supply (4) configured to apply a voltage between the second electrode (5) and the second counter electrode (7);
    an air-sending device (1) introducing air into the air path housing (10), and **characterised by**:

    a member (9, 15) that insulates the hydrophilic filter (6) from the second electrode (5) and the second counter electrode (7); and
    humidifying means (19) configured to supply the charged airborne microorganisms with moisture and disposed on the leeward side of the first electrode (2) and the first counter electrode (3)"
    wherein moisture supplied by the humidifying means is mixed with the charged airborne microorganisms and the airborne microorganisms are captured by the hydrophilic filter (6) and inactivated.

2. The apparatus (100) for capture and inactivation of microbes and viruses of claim 1, wherein the airborne microorganisms are inactivated after being captured by the hydrophilic filter (6) and charged.

3. The apparatus (100) for capture and inactivation of microbes and viruses of claim 2, wherein the air path housing (10) is configured to be closed by an opening/closing device when the airborne microorganisms are inactivated.

4. The apparatus (100) for capture and inactivation of microbes and viruses of claim 3, further comprising an opening and closing device for opening and closing the air path housing (10), wherein
the opening and closing device opens the air path housing (10) when the air is sent by the air-sending device (1) and closes the air path housing (10) when the air-sending device (1) is stopped.

5. The apparatus (100) for capture and inactivation of microbes and viruses of claim 2, further comprising:

an opening and closing device for opening and closing the air path housing (10),
a bypass (44) connecting a downstream side of the air-sending device (1) with a downstream side of the hydrophilic filter (6), wherein
the air in the air path housing (10) is circulated via the bypass (44) in the process of inactivating the airborne microorganisms.

**Patentansprüche**

1. Vorrichtung (100) zur Erfassung und Deaktivierung von Mikroben und Viren, umfassend:

ein Luftpfadgehäuse (10);
eine erste Elektrode (2), konfiguriert, mit einer Spannung versehen zu werden, um luftgetragene Mikroorganismen aufzuladen, die in das Luftpfadgehäuse (10) eingeführt werden;
eine erste Gegenelektrode (3), die so angeordnet ist, dass sie der ersten Elektrode (2) zugewandt ist;
eine Hochspannungsstromversorgung (8), konfiguriert, eine Spannung zwischen der ersten Elektrode (2) und der ersten Gegenelektrode (3) vorzusehen;
einen hydrophilen Filter (6), der die luftgetragenen Mikroorganismen erfasst, die durch eine zwischen der ersten Elektrode (2) und der ersten Gegenelektrode (3) erzeugten Entladung aufgeladen werden;
eine zweite Elektrode (5), konfiguriert, den hydrophilen Filter (6) einer elektrostatischen Induktion zu unterziehen;
eine zweite Gegenelektrode (7), die so angeordnet ist, dass die der zweiten Elektrode (5) zugewandt ist, wobei der hydrophile Filter (6) dazwischen liegt;
eine Hochspannungsstromversorgung (4), konfiguriert, eine Spannung zwischen der zweiten Elektrode (5) und der zweiten Gegenelektrode (7) vorzusehen;
eine Luftsendevorrichtung (1), die Luft in das Luftpfadgehäuse (10) einführt und **gekennzeichnet durch**:

ein Element (9, 15), das den hydrophilen Filter (6) von der zweiten Elektrode (5) und der zweiten Gegenelektrode (7) isoliert; und
Befeuchtungsmittel (19), konfiguriert, Feuchtigkeit an die aufgeladenen luftgetragenen Mikroorganismen zu liefern und angeordnet auf der Leeseite der ersten Elektrode (2) und der ersten Gegenelektrode (3), wobei die vom Befeuchtungsmittel gelieferte Feuchtigkeit mit den aufgeladenen luftgetragenen Mikroorganismen vermischt wird und die luftgetragenen Mikroorganismen vom hydrophilen Filter (6) erfasst und deaktiviert werden.

2. Vorrichtung (100) zur Erfassung und Deaktivierung von Mikroben und Viren nach Anspruch 1, wobei die luftgetragenen Mikroorganismen deaktiviert werden, nachdem sie vom hydrophilen Filter (6) erfasst und aufgeladen wurden.

3. Vorrichtung (100) zur Erfassung und Deaktivierung von Mikroben und Viren nach Anspruch 2, wobei das Luftpfadgehäuse (10) konfiguriert ist, durch eine Öffnungs-/Schließvorrichtung geschlossen zu werden, wenn die luftgetragenen Mikroorganismen deaktiviert werden.

4. Vorrichtung (100) zur Erfassung und Deaktivierung von Mikroben und Viren nach Anspruch 3, ferner umfassend eine Öffnungs- und Schließvorrichtung zum Öffnen und Schließen des Luftpfadgehäuses (10), wobei die Öffnungs- und Schließvorrichtung das Luftpfadgehäuse (10) öffnet, wenn die Luft von der Luftsendevorrichtung (1) gesendet wird und das Luftpfadgehäuse (10) schließt, wenn die Luftsendevorrichtung (1) angehalten wird.

5. Vorrichtung (100) zur Erfassung und Deaktivierung von Mikroben und Viren nach Anspruch 2, ferner umfassend eine Öffnungs- und Schließvorrichtung zum Öffnen und Schließen des Luftpfadgehäuses (10),

einen Bypass (44), der eine stromabwärtige Seite der Luftsendevorrichtung (1) mit einer stromabwärtigen Seite des hydrophilen Filters (6) verbindet, wobei

die Luft im Luftpfadgehäuse (10) mittels des Bypass (44) beim Verfahren des Deaktivierens der luftgetragenen Mikroorganismen zirkuliert wird.

## Revendications

1. Appareil (100) de capture et d'inactivation de microbes et de virus, comprenant :

   un compartiment de passage d'air (10) ;
   une première électrode (2) conçue pour recevoir l'application d'une tension permettant de charger des micro-organismes aéroportés introduits dans le compartiment de passage d'air (10) ;
   une première contre-électrode (3) située face à la première électrode (2) ;
   une alimentation de haute tension (8) conçue pour appliquer une tension entre la première électrode (2) et la première contre-électrode (3) ;
   un filtre hydrophile (6) qui capture les micro-organismes aéroportés chargés par la décharge générée entre la première électrode (2) et la première contre-électrode (3) ;
   une deuxième électrode (5) conçue pour soumettre le filtre hydrophile (6) à une induction électrostatique ;
   une deuxième contre-électrode (7) située face à la deuxième électrode (5), le filtre hydrophile (6) étant intercalé entre les deux ;
   une alimentation en haute tension (4) conçue pour appliquer une tension entre la deuxième électrode (5) et la deuxième contre-électrode (7) ;
   un dispositif d'envoi d'air (1) introduisant de l'air dans le compartiment de passage d'air (10), et **caractérisé par** :

   un élément (9, 15) qui isole le filtre hydrophile (6) de la deuxième électrode (5) et de la deuxième contre-électrode (7) ; et
   un moyen d'humidification (19) conçu pour apporter de l'humidité aux les micro-organismes aéroportés chargés, et situé sur le côté sous le vent de la première électrode (2) et de la première contre-électrode (3), l'humidité apportée par le moyen d'humidification étant mélangée aux micro-organismes aéroportés chargés et les micro-organismes aéroportés étant capturés par le filtre hydrophile (6) et inactivés.

2. Appareil (100) de capture et d'inactivation de microbes et de virus selon la revendication 1, dans lequel les micro-organismes aéroportés sont inactivés après avoir été capturés par le filtre hydrophile (6) et chargés.

3. Appareil (100) de capture et d'inactivation de microbes et de virus selon la revendication 2, dans lequel le compartiment de passage d'air (10) est conçu pour être fermé par un dispositif d'ouverture/fermeture lorsque les micro-organismes aéroportés sont inactivés.

4. Appareil (100) de capture et d'inactivation de microbes et de virus selon la revendication 3, comprenant en outre un dispositif d'ouverture et de fermeture destiné à ouvrir et fermer le compartiment de passage d'air (10), dans lequel le dispositif d'ouverture et de fermeture ouvre le compartiment de passage d'air (10) lorsque l'air est envoyé par le dispositif d'entrée d'air (1) et ferme le compartiment de passage d'air (10) lorsque le dispositif d'entrée d'air (1) est arrêté.

5. Appareil (100) de capture et d'inactivation de microbes et de virus selon la revendication 2, comprenant en outre :

   un dispositif d'ouverture et de fermeture destiné à ouvrir et fermer le compartiment de passage d'air (10),
   une déviation (44) reliant un côté aval du dispositif d'envoi d'air (1) à un côté aval du filtre hydrophile (6), et
   l'air présent dans le compartiment de passage d'air (10) circule par la déviation (44) lors du processus d'inactivation des micro-organismes aéroportés.

F I G. 1

F I G. 2

F I G.  3

START PROCESS OF
CAPTURING VIRUSES

S101 — START APPLYING VOLTAGES TO
CHARGING UNIT AND CAPTURING UNIT

S102 — DISCHARGE CURRENT IN CHARGING
UNIT IS IN PROPER RANGE?    NO

YES

S103 — CHARGE VIRUSES BY DISCHARGE

S104 — CAPTURE CHARGED VIRUSES WITH
ELECTROSTATIC INDUCTION

S105 — PROCESSING TIME IS
ELAPSED?    NO

YES

S106 — FINISH PROCESS OF
CAPTURING VIRUSES

S107 — START PROCESS OF INAC-
TIVATING VIRUSES

S108 — DISCHARGE IN VIRUS
CAPTURING UNIT

S109 — PROCESSING TIME IS
ELAPSED?    NO

YES

S110 — FINISH PROCESS OF
INACTIVATING VIRUSES

S111 — START PROCESS OF CAP-
TURING VIRUSES

22

F I G. 4

F I G. 5

FIG. 6

FIG. 7

FIG. 8

FIG. 9

F I G. 1 0

7  6  5  4  3  12  1

10  9  8

100b

F I G. 1 1

7  6  5  4  3  13  1

10  9  8

100c

FIG. 12

FIG. 13

FIG. 14

FIG. 15

FIG. 16

FIG. 17

FIG. 18

FIG. 19

FIG. 20

CROSS-SECTION
TAKEN ALONG A-A'

FIG. 21

F I G. 2 2

F I G. 2 3

F I G.  2 4

CLEANED AIR

AIR CONTAINING
VIRUSES AND
MICROBES

F I G.  2 5

CLEANED AIR

AIR CONTAINING
VIRUSES AND
MICROBES

FIG. 26

FIG. 27

FIG. 28

FIG. 29

F I G. 3 0

FIG. 31

FIG. 32

F I G. 3 3

F I G. 3 4

F I G. 3 5

F I G . 3 6

F I G . 3 7

CHARGING/INACTIVATING
POWER SUPPLY

POSITIVE
RECTIFIER

HIGH VOLTAGE
POWER SUPPLY

NEGATIVE
RECTIFIER

CHARGING/
INACTIVATING
ELECTRODE

SWITCH

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007512131 W
- JP 11188214 A
- US 2008170971 A1
- JP 1249145 A
- JP 2010022998 A

- JP 2010029865 B
- JP 2006043550 A
- EP 1980317 A1
- JP 2005304762 A